(19)

**Europäisches Patentamt**
**European Patent Office**
**Office européen des brevets**

(11) **EP 3 956 355 B1**

(12) **EUROPEAN PATENT SPECIFICATION**

(45) Date of publication and mention
of the grant of the patent:
**26.11.2025 Bulletin 2025/48**

(21) Application number: **20791191.8**

(22) Date of filing: **17.04.2020**

(51) International Patent Classification (IPC):
***C07K 14/62*** *(2006.01)* ***A61K 38/17*** *(2006.01)*
***A61P 3/08*** *(2006.01)*

(52) Cooperative Patent Classification (CPC):
**A61P 3/08; C07K 14/62;** A61K 38/00

(86) International application number:
**PCT/US2020/028770**

(87) International publication number:
**WO 2020/214955 (22.10.2020 Gazette 2020/43)**

(54) **STABILIZATION OF PRANDIAL OR BASAL INSULIN ANALOGUES BY AN INTERNAL DISELENIDE BRIDGE**

STABILISIERUNG VON PRANDIALEN ODER BASALEN INSULINANALOGA DURCH EINE INTERNE DISELENIDBRÜCKE

STABILISATION D'ANALOGUES D'INSULINE PRANDIALE OU BASALE PAR UN PONT DISÉLÉNIURE INTERNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.04.2019 US 201962836264 P**

(43) Date of publication of application:
**23.02.2022 Bulletin 2022/08**

(73) Proprietors:
• **The Trustees of Indiana University**
**Bloomington, IN 47405 (US)**
• **Yissum Research and Development Company of the**
**Hebrew University of Jerusalem Ltd.**
**9139002 Jerusalem (IL)**

(72) Inventors:
• **METANIS, Norman**
**14930 Maghar (IL)**
• **KTORZA, Orit**
**9372110 Jerusalem (IL)**
• **SHOHAM, Gil**
**90805 Jerusalem (IL)**
• **WEISS, Michael A.**
**Indianapolis, Indiana 46204 (US)**

(74) Representative: **HGF**
**HGF Limited**
**1 City Walk**
**Leeds LS11 9DX (GB)**

(56) References cited:
**WO-A1-2018/045018    WO-A2-2016/172269**
**US-A1- 2005 039 235    US-A1- 2018 118 801**

• **ORIT WEIL-KTORZA ET AL: "Substitution of an Internal Disulfide Bridge with a Diselenide Enhances both Foldability and Stability of Human Insulin", CHEMISTRY - A EUROPEAN JOURNAL, JOHN WILEY & SONS, INC, DE, vol. 25, no. 36, 16 May 2019 (2019-05-16), pages 8513 - 8521, XP071849593, ISSN: 0947-6539, DOI: 10.1002/CHEM.201900892**
• **BALAMURUGAN DHAYALAN ET AL: "Reassessment of an Innovative Insulin Analogue Excludes Protracted Action yet Highlights the Distinction between External and Internal Diselenide Bridges", CHEMISTRY - A EUROPEAN JOURNAL, JOHN WILEY & SONS, INC, DE, vol. 26, no. 21, 18 March 2020 (2020-03-18), pages 4695 - 4700, XP071851216, ISSN: 0947-6539, DOI: 10.1002/CHEM.202000309**

**(Cont. next page)**

- **ARAI ET AL.: "Preparation of Selenoinsulin as a Long-Lasting Insulin Analogue", ANGEW CHEM INT ED ENGL., vol. 129, 2017, pages 5614 - 5618, XP055672085**

Remarks:

The complete document including Reference Table(s) and the Sequence Listing(s) can be downloaded from the EPO website

**Description**

**BACKGROUND**

Insulin is a peptide hormone comprised of a two-chain heterodimer that is biosynthetically derived from a low-potency single-chain proinsulin precursor through enzymatic processing. Human insulin is comprised of two peptide chains (an "A chain" (SEQ ID NO: 2) and "B chain" (SEQ ID NO: 4)) bound together by disulfide bonds and having a total of 51 amino acids (aa). The A chain and B chain associate in a three-dimensional structure to assemble contact surfaces to enable high-affinity binding to the insulin receptor. These surfaces in part reorganize on receptor binding relative to the free hormone.

Insulin and its analogues demonstrate unparalleled ability to lower the concentration of glucose in the blood via cellular entry. An abnormal increase in blood-glucose concentration is a key issue in virtually all forms of diabetes mellitus (DM). Wild-type (WT) human insulin and other animal insulin molecules (as encoded in the genomes of other vertebrates) bind to insulin receptors (IRs) in multiple organs and diverse types of cells, irrespective of the receptor isoform generated by alternative modes of RNA splicing (designated isoform A or B; IR-A or IR-B) and further elaborated by patterns of post-translational glycosylation, which may be tissue-specific. The pharmacokinetic and pharmacodynamic (PK/PD) properties of WT human insulin and other animal insulins are often suboptimal in subsets of patients with DM. The associated unmet medical needs have led over the past three decades to the design, development and clinical use of insulin analogues (Sharma, A.K., et al., 2019). The two major classes of insulin analogue formulations are (i) those that are absorbed more rapidly from a subcutaneous depot (and so faster acting) than are regular human insulin formulations (Nørgaard, K., et al., 2018) and (ii) those that are absorbed less rapidly (and so slower acting and more prolonged) than are micro-crystalline suspensions of protamine-associated WT human insulin (Hompesch, M., et al. 2019). These classes are respectively designated as prandial analogue formulations or basal analogue formulations.

Unfortunately, prandial and basal insulin analogue formulations in current clinical use are typically more susceptible to chemical and physical degradation at or above room temperature than are formulations containing WT human insulin, especially rapid-acting insulin analogue formulations as used in insulin pumps (Woods, R.J., *et al.*, 2012). Indeed, only one clinical insulin analogue formulation known in the art exhibits a shelf life stability greater than that of WT insulin (basal product Tresiba®; Novo-Nordisk). Accordingly, there is a continuing need for therapeutic insulin analogues that exhibit enhanced pharmaceutical properties, such as increased thermodynamic stability and augmented resistance to thermal fibrillation at or above room temperature.

It would therefore be of medical benefit to augment the thermodynamic stability of an insulin analogue (or any therapeutic protein) and particularly to mitigate its susceptibility to physical degradation (fibrillation). Administration of insulin in some form (an animal insulin, human insulin or human insulin analogue) has long been established as a treatment for DM. A major goal of conventional insulin replacement therapy in patients with DM is tight control of the blood-glucose concentration to prevent its excursion above or below the normal range characteristic of healthy human subjects. Excursions below the normal range are associated with immediate adrenergic or neuroglycopenic symptoms, which in severe episodes can lead to convulsions, coma, and death. Excursions above the normal range are associated with increased long-term risk of microvascular disease (including retinapathy, blindness and renal failure) and probably also the long-term risk macrovascular disease. Critical to the safe and convenient achievement of tight glycemic control by patients with Type 1 DM and by a subset of patients with Type 2 DM has been the development of novel insulin analogues that differ in sequence from naturally occurring mammalian insulins due to the presence of amino-acid substitutions or modified amino-acid side chains. Such substitutions and modifications have been introduced in the art to make canonical "regular" WT insulin formulations (soluble formulations of zinc-stabilized hexamers at neutral pH as known in the art) even more rapid and likewise to make canonical long-acting WT insulin formulations (typically microcrystalline suspensions) even longer acting. Despite their favorable PK/PD properties, such insulin analogue formulations may exhibit reduced thermodynamic stability and/or increased susceptibility to fibrillation, limiting shelf life at or above room temperature.

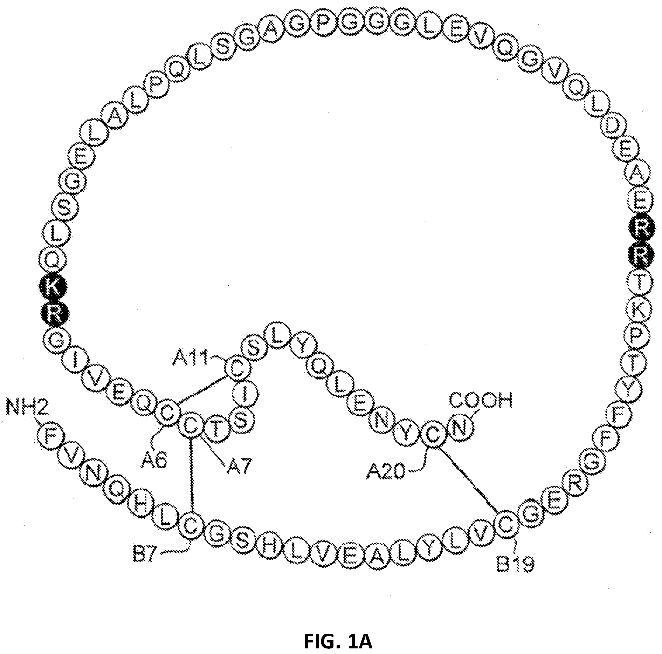

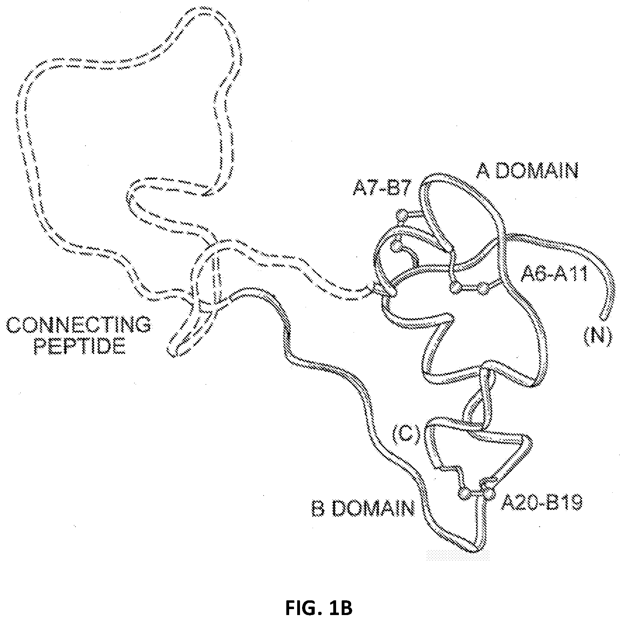

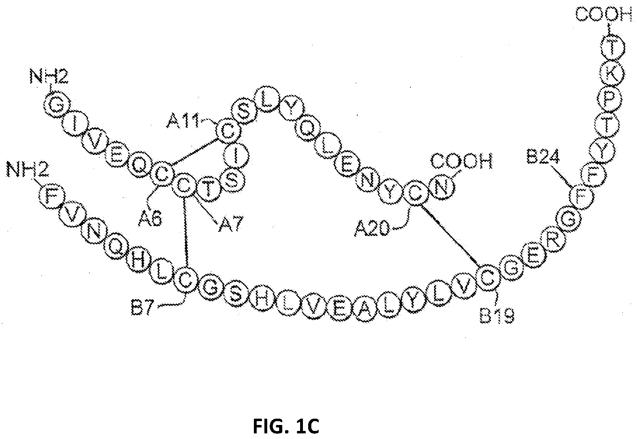

Insulin is a small globular protein that plays a central role in regulating the metabolism of vertebrates. Insulin is stored in the secretory granules of pancreatic $\beta$-cells as $Zn^{2+}$-stabilized hexamers, but functions as a $Zn^{2+}$-free monomer in the bloodstream. Insulin is the product of a single-chain precursor, proinsulin, in which a connecting region (35 residues) links the C-terminal residue of B chain (residue B30) to the N-terminal residue of the A chain (Fig. 1A). A variety of evidence indicates that the three-dimensional (3D) structure of proinsulin consists of an insulin-like core and disordered connecting peptide (Fig. 1B). Formation of three specific disulfide bridges (pairings A6-A11, A7-B7, and A20-B19; Fig. 1A and 1B) is thought to be coupled to oxidative folding of proinsulin in the rough endoplasmic reticulum (ER). Proinsulin assembles to form soluble $Zn^{2+}$-coordinated hexamers shortly after export from the ER to the Golgi apparatus. Endoproteolytic digestion and conversion to insulin (Fig. 1C) occurs in immature secretory granules followed by morphological condensation. Crystalline arrays of zinc insulin hexamers within mature storage granules have been visualized in mouse islets by electron microscopy (EM).

The sequence of proinsulin (with component A and B domains) is shown in schematic form in Fig. 1A. Individual residues are indicated by the identity of the amino acid (typically using a standard three-letter code), the chain and sequence position (typically as a superscript). Pertinent to the present disclosure are the conserved cysteine residues at positions A6 and A11 ($Cys^{A6}$ and $Cys^{A11}$). These sulfur-containing side chains pair to form an internal disulfide bridge. The atoms of this bridge are not in contact with the insulin receptor (IR), as visualized in structures of insulin bound to fragments of the IR by X-ray crystallography and cryo-electron microscopic image reconstruction. Formation of the A6-A11 bridge (also designated cystine A6-A11) does, however, make a critical contribution to the packing of the hydrophobic core of insulin and to its thermodynamic stability.

The present disclosure is directed to insulin analogue formulations having (a) increased shelf life at or above room temperature and (b) in the case of prandial analogues, increased stability within the reservoir of an insulin pump. The latter is important to prevent pump occlusion and potential life-threatening diabetic ketoacidosis. Because WT human insulin formulations are ordinarily stabilized by zinc ions (which promote zinc-coordinated hexamer assembly), these needs are especially prominent in the context of amino-acid substitutions that are known in the art either to (i) reduce the stability of the zinc insulin hexamer (as exemplified by insulin *lispro* and insulin *aspart;* respectively the active components of Humalog® [Lilly] and Novolog® [Novo-Nordisk]), (ii) be used in zinc-free formulations (as exemplified by insulin *glulisine,* the active component of Apidra® [Sanofi]), (iii) be used in zinc-containing formulations at acidic pH wherein zinc ions do not bind to the protein and so do not promote protective hexamer assembly (as exemplified by insulin *glargine,* the active component of Lantus® and Toujeo® [Sanofi]). Restrictions regarding the storage and use of such products at or above room temperature have been imposed by the U.S. Food & Drug Administration as a result of these properties.

Previous efforts to mitigate the instability of insulin analogue formulations have employed conventional amino-acid substitutions on the surface of insulin, including introduction of one or more additional disulfide bridges spanning a surface not engaged in receptor binding. The utility of such surface substitutions or modifications has been confounded by unexpected and undesirable changes in receptor-binding properties or in signaling properties, such as increased mitogenicity leading to elevated risk of cancer on chronic administration of the insulin analogue (in the case of the stabilizing $Asp^{B10}$ substitution on the surface of the B chain) or aberrantly prolonged cellular signaling (in the case of an engineered disulfide bridge between residues B4 and A10). The utility of additional disulfide bridges has thus been confounded by unexpected and undesirable changes in biological properties, such as prolonged signaling once the IR is engaged, also creating concern for induction of cancer. Because of the evolutionary conservation of the core of insulin and its structural elegance, conventional amino-acid substitutions in the interior of insulin are generally associated with decreased themodynamic stability; to our knowledge, no such core substitution has been identified with enhanced stability.

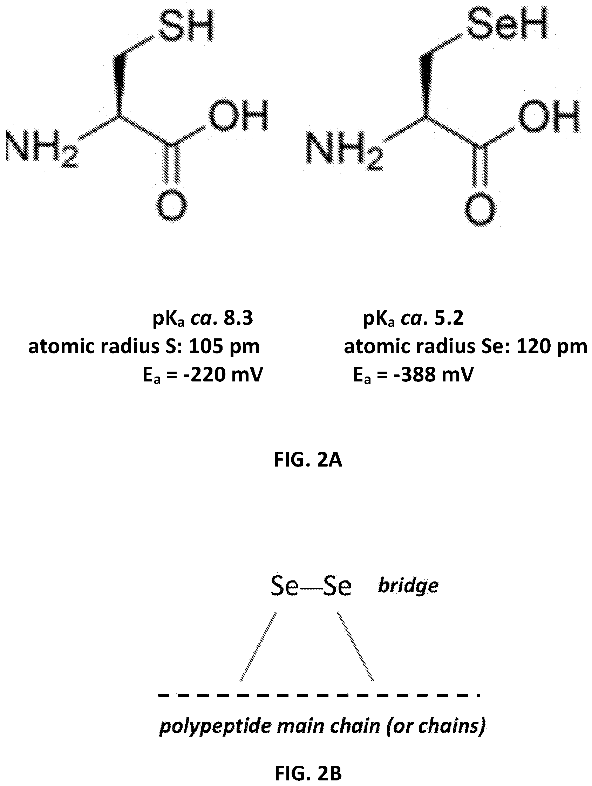

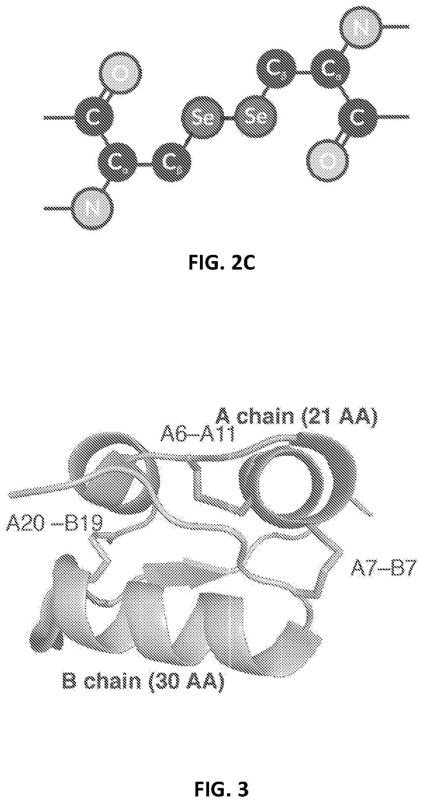

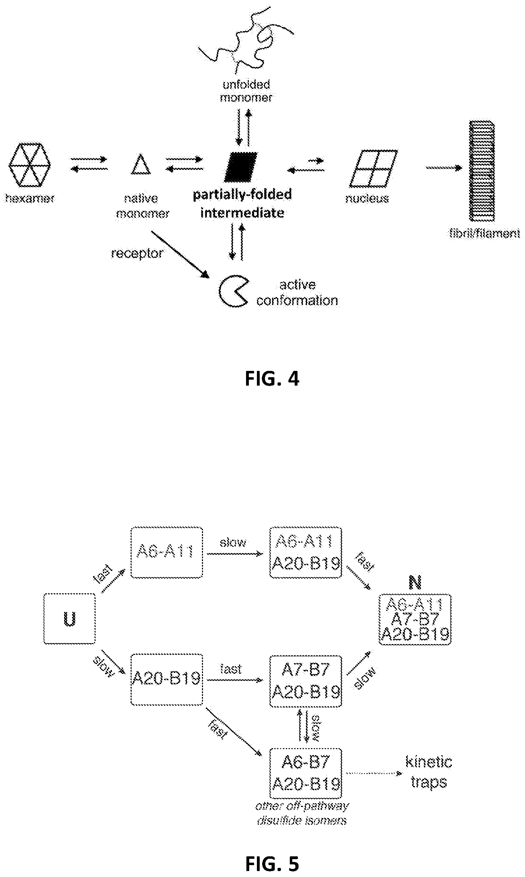

The challenge of finding modifications that forestall fibrillation has been magnified by the lack of a consistent association between the free energy of unfolding ($\Delta G_U$) and the lag time prior to onset of fibrillation. The present invention exploits the distinctive physico-chemical properties of selenio-cysteine relative to cysteine (Sec *versus* Cys; Figs. 2A). Just as two Cys residues may pair to form a disulfide bridge (cystine), two Sec residues may pair to form a diselenide bridge (Figs. 2B and 2C). A surprising aspect of the present disclosure is that pairwise substitution of the sulfur atoms in residues A6 and A11 in the interior of a rapid-acting insulin analogue or in the interior of a basal insulin analogue by selenium atoms should enhance stability and forestall fibrillation without change in receptor-binding affinity. The location of cystine A6-A11 is shown in Fig. 3; a mechanistic scheme describing the pathway of insulin fibrillation is shown in Fig. 4. The present disclosure thus views the chemistry of selenocysteine and selenocystine from a perspective unrelated to traditional views of oxidation-reduction chemistry as known in the art (Beld, J. *et al.* 2007, 2008, 2010).

The baseline instability of WT animal insulins and their intrinsic susceptibility to chemical and physical degradation in a pharmaceutical formulation has generally been mitigated through the inclusion of zinc ions, which promote the native assembly of $Zn^{2+}$-coordinated insulin hexamers. Such hexamers contain two axial zinc ions in their interior and exhibit greater stability than zinc-free insulin solutions. Zinc-containing insulin formulations generally exhibit shelf lives than corresponding formulations lacking zinc ions (Brange, J. (1987) in *Galenics of Insulin,* Springer, Berlin, Germany [https://doi.org/10.1007/978-3-662-02526-0_3]). The requirement for zinc ions is exacerbated by the amino-acid substitutions in certain insulin analogues in current clinical use (such as insulin *lispro* and insulin *aspart*) due to their lower thermodynamic stabilities and increased susceptibility to fibrillation. It would therefore be desirable to enhance the intrinsic stability of insulin analogues and their resistance to fibrillation such that pharmaceutical formulations could include excipients that could favorably enhance the speed or efficiency of insulin absorption from a subcutaneous depot but would compete with insulin for the binding of zinc ions. Examples of such excipients known in the art include triphosphate ions, trisulfate ions, and divalent-ion chelating agents, including ethylene-diamine-tetra-acetic acid (EDTA) and ethylene glycol-bis(β-aminoethyl ether)-N,N,N',N'-tetraacetic acid (EGTA).

It would be desirable, therefore, to provide a novel class of rapid-acting and long-acting insulin analogues whose monomeric structure is stabilized on a thermodynamic basis, such that global unfolding is minimized in the conformational folding equilibrium and such that subglobal conformational fluctuations are also damped. Enhanced stability and damped fluctuations are respectively associated with decreased rates of chemical degradation and physical degradation, together leading to an extended shelf life at or above room temperature. More generally, there is a need for a molecular design strategy to stabilize the insulin molecule without altering its receptor-binding surfaces.

Applicant has met that need by providing insulin analogues containing pairwise substitution of cysteines at position A6 and A11 by selenocysteine (Sec). The latter amino acid (which occurs naturally in the human body and is considered as the "21st encoded amino acid") contains a single atomic substitution: replacement of the sulfur atom in the side-chain thiol group by selenium. Pairs of Sec residues may form diselenide bridges, which are similar to disulfide bridges but with certain changes in physico-chemical properties. Pairwise Cys→Sec substitutions in a polypeptide may be favorable or unfavorable depending on the structural environment of the particular residues. The present application discloses that a specific diselenide bridge in a rapid-acting insulin analogue or a basal insulin analogue will be useful in enhancing their pharmacologic properties with respect to shelf life at or above room temperature, *i.e.,* to mitigate the susceptibility of such protein formulations to chemical and physical degradation.

## SUMMARY

In accordance with the present disclosure, insulin analogues are provided wherein a pairwise substitution of the cysteines at positions corresponding to A6 and A11 of native insulin have been replaced by selenocysteine. In one embodiment an analogue is a rapid-acting or long-acting insulin analogue that comprises a pairwise substitution of the cysteines at positions corresponding to A6 and A11 of native insulin. This pairwise substitution has been discovered to stabilize the 3D structure of the protein and/or delay the onset of fibrillation. The A6 and A11 positions in the A chain of mammalian insulins (and indeed in all vertebrate insulins, proinsulins and insulin-like growth factors, including IGF-I, IGF-II and relaxin family members) are conserved as cysteine. The two cysteines pair to form cystine A6-A11, one of three disulfide bridges in the vertebrate insulin superfamily. In 3D structures of the zinc-free insulin dimer and zinc insulin hexamers, the A6-A11 disulfide bridge packs within a cluster of aromatic and aliphatic side chains within the hydrophobic core of an individual monomer (Fig. 3). Although not wishing to be restricted by theory, gaps between side chains occur within this cluster such that atoms slightly larger than sulfur might be accommodated without nonlocal structural perturbations-and indeed a "sweet spot" of atomic radii might exist such that a slightly larger atom than sulfur would improve the efficiency of side-chain packing within this cluster.

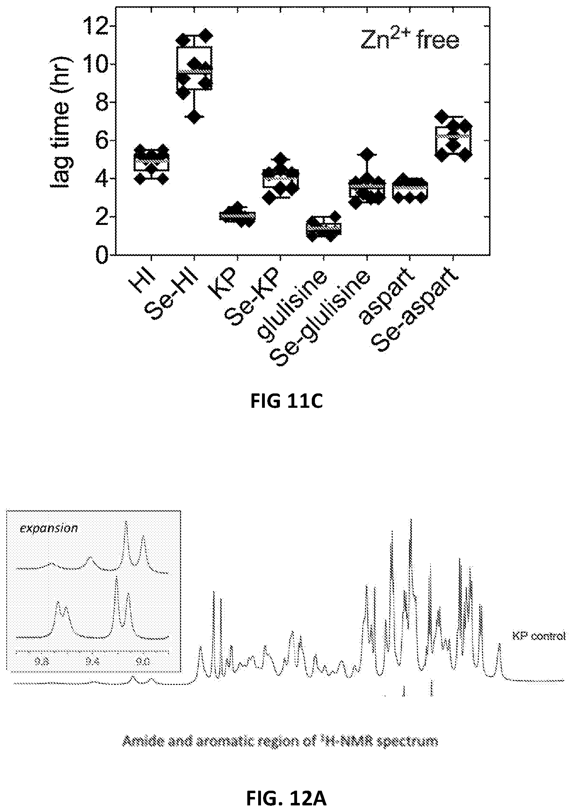

As disclosed herein, pairwise substitution of the two sulfur atoms in cystine A6-A11 by selenium atoms stabilizes WT insulin and diverse insulin analogue molecules (Table 1); in the case of the three prandial insulin analogues in current clinical use (insulin *lispro,* insulin *aspart* and insulin *glulisine*) forestalls its fibrillation at or above room temperature (Table 2 and Fig. 11C, below). Because these paired modifications occur within the core of insulin (and not at its receptor-binding surfaces and not at its self-assembly surfaces), the analogues of the present disclosure are compatible with a broad range of conventional amino-acid modifications to the surface of an insulin monomer and with a broad range of non-peptidic surface modifications (such as acetylation or derivatization by carbohydrates, polyethylene glycol or phenyl-boronic acids) that are known in the art to alter the assembly, PK, PD or clearance properties of insulin analogue formulations.

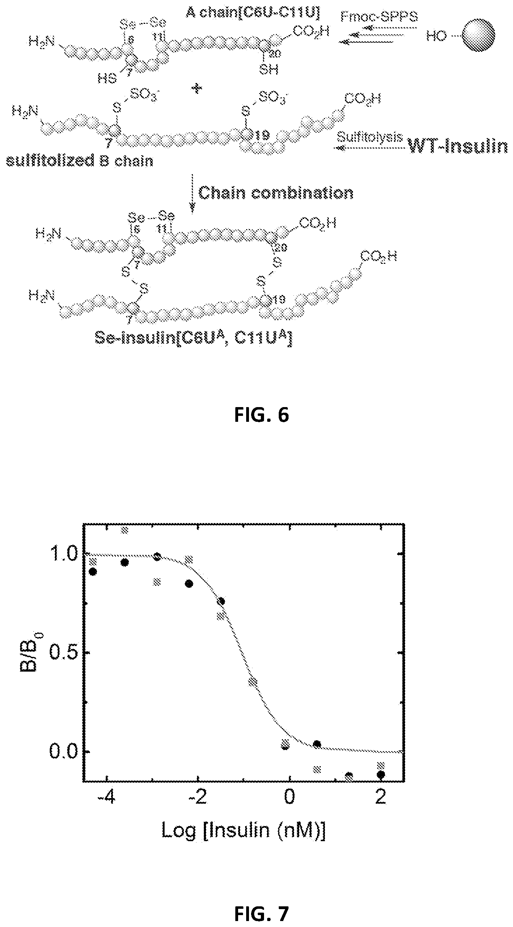

In another embodiment a rapid-acting or long-acting insulin analogue is provided that contains a diselenide bridge between residues A6 and A11 that extends the shelf life of the active insulin receptor agonist in a pharmaceutical formulation for the treatment of diabetes mellitus. A further aspect of the present invention holds that the aforementioned pharmaceutical formulations may, in the case of rapid-acting formulations, not require inclusion of zinc ions (known in the art to enhance the stability of insulin analogues and promote its native self-assembly) and thereby enable inclusion of a broad range of excipients that would otherwise interact with or chelate zinc ions, including excipients intended to accelerate the absorption of insulin from a subcutaneous depot. It is yet another aspect of the present analogues that the A6 and A11 paired Sec substitutions provides a method to enhance the speed and fidelity of insulin chain combination (Kung, Y.T., et al., 1965; Katsoyannis, P.G., 1966), especially in the context of B-chain variants known to impair this reaction in combination with the WT A chain. A mechanistic scheme for insulin chain combination is shown in Fig. 5; its enhancement via pairwise substitutions Cys$^{A6}$→Sec and Cys$^{A11}$→Sec is depicted in Fig. 6.

In one embodiment an insulin analogue is provided wherein the insulin A chain comprises the sequence GIVEQX$_6$CX$_8$SIX$_{11}$SLYQLENYCX$_{21}$-R$_{53}$ (SEQ ID NO: 15) and the insulin B chain comprises the sequence FVX$_{23}$QX$_{25}$LCGSHLVEALYLVCGERGFFYTX$_{48}$X$_{49}$X$_{50}$ (SEQ ID NO: 16), wherein

X$_6$ is selenocysteine;
X$_8$ is histidine, threonine, lysine, arginine, phenylalanine or tryptophan;
X$_{11}$ is selenocysteine; and
X$_{21}$ is selected from the group consisting of alanine, glycine, serine, threonine, glutamine, asparagine, and aspartic acid;
X$_{23}$ is Asn or Lys:

X$_{25}$ is histidine or threonine;
X$_{48}$ is proline, lysine or aspartic acid;

$X_{49}$ is proline, lysine or glycine;
$X_{50}$ is threonine, alanine or serine; and
$R_{53}$ is COOH or $CONH_2$.

In one embodiment an insulin analogue is provided wherein the A chain comprises the sequence of GIVEQX$_6$CT-SIX$_{11}$SLYQLENYCN (SEQ ID NO: 30) and the B chain comprises the sequence FVNQHLCGSHLVEALYLVCGERGFFYTPKT (SEQ ID NO: 4), wherein $X_6$ and $X_{11}$ are both selenocysteine.

In accordance with one embodiment the insulin analogue is a two-chain analogue wherein the A and B chains are joined to one another by disulfide (S-S) and/or diselenide (Se-Se) covalent bonds.

The solubility and retention times of the A6-A11 diselenide bridged insulin analogues disclosed herein can be modified by the attachment of hydrophilic moieties and/or long-chain hydrocarbons such as fatty acid chains. In one embodiment the A6-A11 diselenide bridged insulin analogues are modified by the covalent linkage of a hydrophilic moiety at one or more positions corresponding to A14, A15, B0, B1, B10, B22, B28, B29. In one embodiment the hydrophilic moiety is albumin, including for example, albumins such as human serum albumin (HSA) and recombinant human albumin (rHA). In one embodiment the hydrophilic moiety is a polyethylene glycol (PEG) chain, having a molecular weight selected from the range of about 500 to about 40,000 Daltons. In one embodiment the polyethylene glycol chain has a molecular weight selected from the range of about 500 to about 5,000 Daltons. In another embodiment the polyethylene glycol chain has a molecular weight of about 10,000 to about 20,000 Daltons.

Acylation or alkylation can increase the half-life of the insulin polypeptides in circulation. Acylation or alkylation can advantageously delay the onset of action and/or extend the duration of action at the insulin receptors. The insulin analogues may be acylated or alkylated at the same amino acid position where a hydrophilic moiety is linked. In one embodiment the acyl group or alkyl group is covalently linked to one or more positions selected from A14, A15, B0, B1, B10, B22, B28, B29 of the insulin peptide. The analogue can also be prepared as dimers or multimers comprising two or more of any of the A6-A11 diselenide bridged insulin analogues disclosed herein either as homodimers/homomultimers or as heterodimers/heteromultimers.

Also encompassed by the present disclosure are pharmaceutical compositions comprising the A6-A11 diselenide bridged insulin analogues disclosed herein, and a pharmaceutically acceptable carrier. In accordance with one embodiment a pharmaceutical composition is provided comprising any of the A6-A11 diselenide bridged insulin analogues disclosed herein, preferably at a purity level of at least 90%, 91%, 92%, 93%, 94%, 95%, 96%, 97%, 98% or 99%, and a pharmaceutically acceptable diluent, carrier or excipient. Such compositions may contain an A6-A11 diselenide bridged insulin analogue as disclosed herein at a concentration of at least 0.5 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml or higher. In one embodiment the pharmaceutical compositions comprise aqueous solutions that are sterilized and optionally stored within various package containers. In other embodiments the pharmaceutical compositions comprise a lyophilized powder. The pharmaceutical compositions can be further packaged as part of a kit that includes a disposable device for administering the composition to a patient. The containers or kits may be labeled for storage at ambient room temperature or at refrigerated temperature.

In accordance with one embodiment an improved method of regulating blood-glucose levels in insulin dependent patients is provided, and more particularly, a method of treating diabetes with a reduced risk of hypoglycemia is provided. The method comprises the steps of administering to a patient an A6-A11 diselenide bridged insulin analogues as disclosed herein in an amount therapeutically effective for the control of diabetes. In one embodiment the A6-A11 diselenide bridged insulin analogues comprises a two-chain prandial insulin analogue comprising an A chain and a B chain linked via interchain disulfide bonds.

**BRIEF DESCRIPTION OF THE DRAWINGS**

Figs. 1A-1C are schematic drawings of human insulin. Fig. 1A is a schematic representation of the sequence of human proinsulin including the A and B chains, and the connecting region, shown with flanking dibasic cleavage sites (filled circles) and C-peptide (open circles). Fig 1B is a structural model of proinsulin, consisting of an insulin-like moiety and a disordered connecting peptide (dashed line). Fig. 1C is a schematic drawing of mature insulin showing the naive disulfide linkages.

Figs. 2A & 2B provide molecular representations of selenocysteine (Sec) *versus* native cysteine (Cys). Fig. 2A highlights the thiol group (-SH) of Cys compared to the -SeH group contained in Sec. Differences in physico-chemical parameters between -SH and -SeH with respect to $pK_a$ (approximately 8.3 for Cys vs approximately 5.2 for Sec) atomic radius (for S, 105 pm vs Se, 120 p,) and $E_o$ -220 mV for Cys vs -388 mV for Sec). Fig. 2B is a schematic representation of a diselenide bridge (-Se-Se-) formed between two selenocysteine residues. Fig. 2C provides a ball-and-stick model of a diselenide bridge.

Fig. 3 presents a ribbon model of wild type (WT) insulin (a single T-state protomer as derived from the crystal structure of a zinc-stabilized hexamer) in which the protein's three disulfide bridges are highlighted: cystine A20-B19, cystine A7-B7 and cystine A6-A11 are indicated. The A-chain ribbon and B-chain ribbon are marked.

Fig. 4 provides a scheme for the mechanism of insulin fibrillation. The protective storage form of insulin as a hexamer is shown at far left, and a protofilament is shown at far right. The horizontal pathway proceeds from the hexamer to the native monomer (triangle), partially folded intermediate (trapezoid), and putative amyloidogenic nucleus (as represented by four conjoined trapezoids). Above and below this pathway are shown the fully denatured state with intact disulfide bridges (top) and the receptor-bound distorted conformation of the insulin monomer (bottom).

Fig. 5 provides a scheme for the mechanism of disulfide pairing in insulin-chain combination. The role of cystine A6-A11 is indicated in the upper pathway. Native pairing can be reached by the upper or lower pathways of the insulin molecule (top) and modifications pertaining to the present disclosure. At bottom are off-pathway steps that may in principle lead to formation of non-native disulfide isomers. Individual steps are labeled "fast" or "slow" in relation to the kinetic properties of these reactions.

Fig. 6 illustrates the expedited mechanism of insulin chain combination envisaged to occur on pair-wise substitution of $Cys^{A6}$ and $Cys^{A11}$ by Sec, leading to rapid initial formation of an A6-A11 diselenide bridge (at top). In the example shown, chemical semi-synthesis of Se-insulin (WT human insulin with $Sec^{A6}$ and $Sec^{A11}$; designated Se-insulin [C6U$^A$, C11U$^A$] employed a synthetic A chain and biosynthetic B chain (obtained from commercial insulin by oxidative sulfitolysis).

Fig. 7 illustrates a receptor-binding assay for insulin receptor (IR) based on competitive displacement of pre-bound $^{125}$I-Tyr$^{A14}$-human insulin. The IR-binding affinity of the Se-insulin analogue (Se-insulin[C6U$^A$, C11U$^A$], ■) was indistinguished from the affinity of a semisynthetic version of WT-insulin (Orn$^{B29}$-insulin, ●). Modeling yielded the estimate $K_d = 0.08 \pm 0.02$ nM.

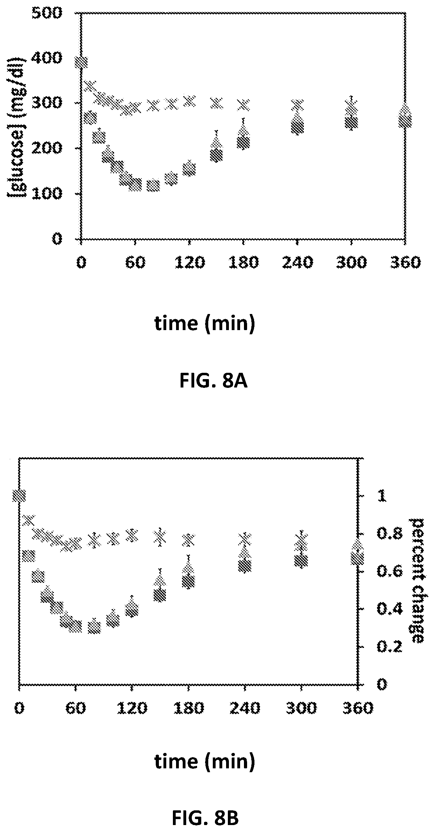

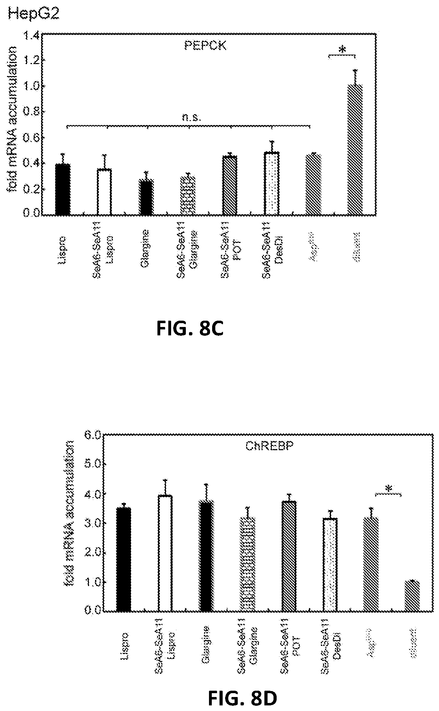

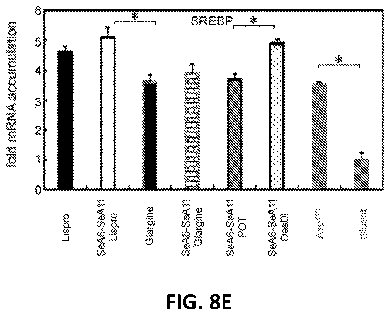

Fig. 8A-8E are graphs presenting data for insulin analogues. Figs. 8A and 8B provide data for two separate functional studies of insulin or insulin analogues in male Sprague-Dawley rats rendered diabetic by β-cell poison streptozotocin (STZ). Comparison of insulin *lispro* (■) *versus* an analogue of insulin *lispro* containing paired substitutions [C6U$^A$, C11U$^A$] (▲), respectively. Injection of protein-free buffer provided a control (X). Nine rats were studied per group. Figs. 8C-8E depicts studies of the biological activity of insulin analogues in cell culture of human liver cell-line HepG2. The studies probed transcriptional responses with respect to genes important in respective pathways of gluconeogenesis or lipid synthesis: phosphoenolpyruvate carboxykinase (PEPCK; gluconeogenesis, Fig. 8C), carbohydrate-responsive element-binding protein (ChREBP; lipid synthesis, Fig. 8D) sterol regulatory element-binding proteins (SREBP; lipid synthesis, Fig. 8E) as analyzed by real-time quantitative reverse-transcriptase polymerase chain reaction (rt-q-rtPCR). The decreased downstream accumulation of PEPCK mRNA and increased accumulation of ChREBP and SREBP mRNA provide readouts for insulin-induced transcriptional signaling in liver metabolism. Brackets designate $p$ values (*) <0.05 or (**) <0.01; "ns" indicates $p$ values > 0.05.

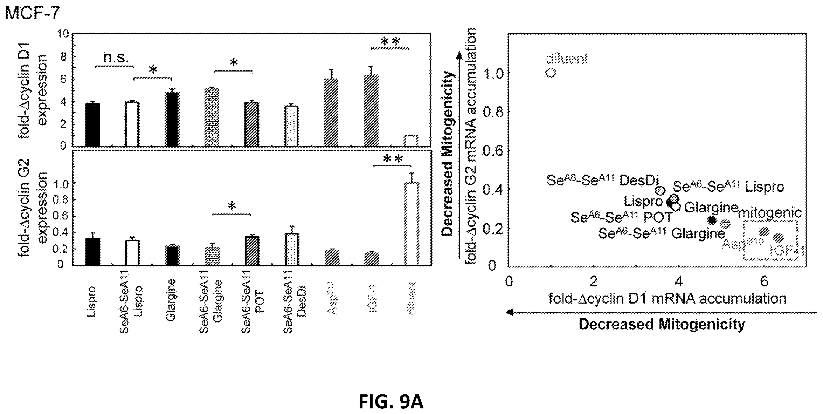

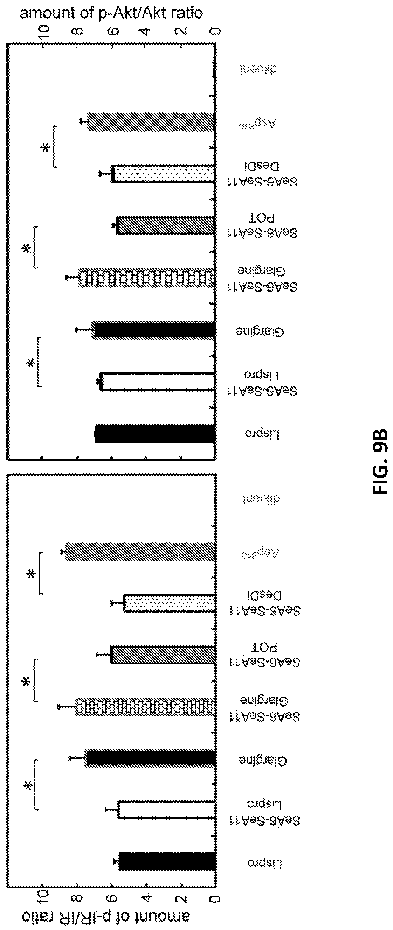

Figs. 9A & 9B document responses of insulin analogues in cell proliferation and insulin receptor signaling in human breast-cancer cell line MCF-7. Fig. 9A contains histograms showing the relative mitogenic signaling (*left*). Brackets designate $p$ values (*) <0.05 or (**) <0.01; "ns" indicates $p$ values > 0.05. Increased expression of the gene encoding cyclin D1 and decreased expression of the gene encoding cyclin G2 are characteristic of cellular proliferation in response to signaling by mitogenic insulin analogues or IGFs. These two-dimensions are simultaneously shown in the panel at *right.* Mitogenic analogue Asp$^{B10}$-insulin (as known in the art) and mitogenic growth factor IGF-1 provided positive controls as highlighted in the "mitogenic zone" of this plot (gray dashed box). Fig. 9B provides histograms describing IR- and Akt signaling as monitored by their respective phosphorylation. Vertical bars provide the average fold-increase over diluent as analyzed by Western blots of p-IR/IR (*left*) and p-Akt/Akt (*right*); * indicates $p$-value < 0.05.

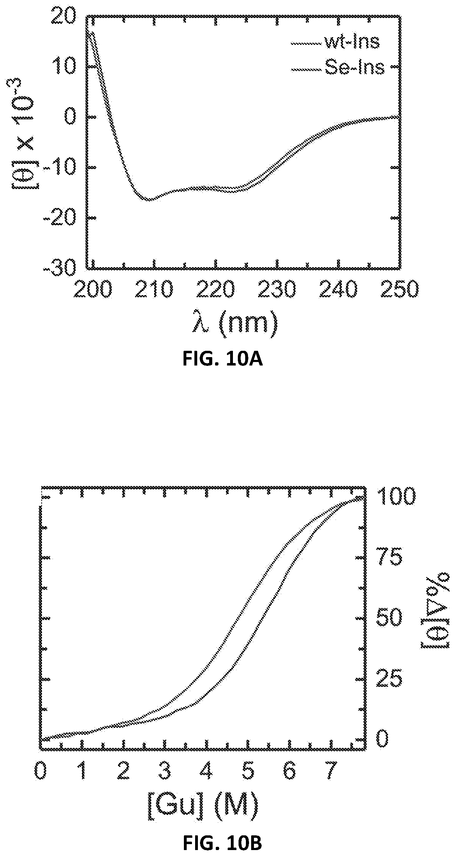

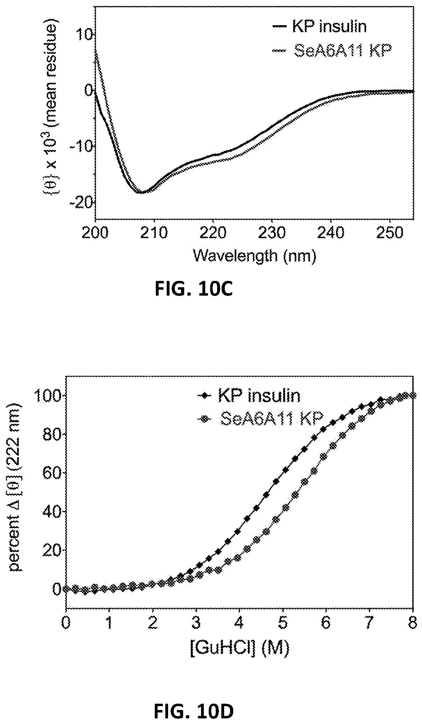

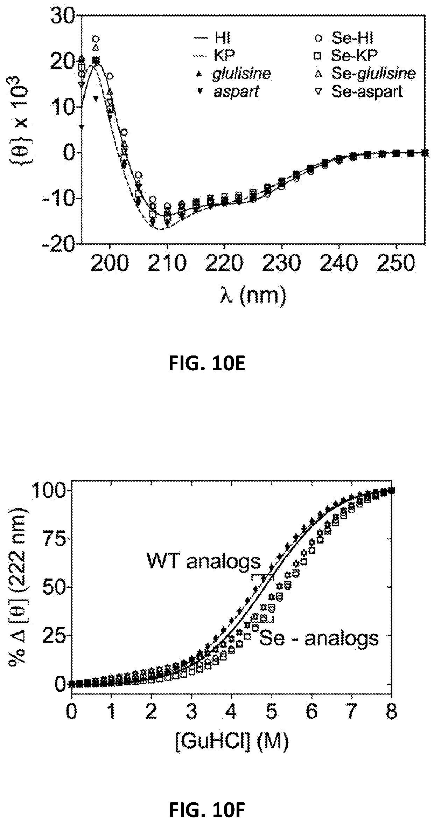

Figs. 10A-10F are graphs presenting data for far-ultraviolet CD spectra and CD-detected chemical denaturation studies for wild type and various insulin analogs. Fig. 10A presents a comparison of CD spectra of WT human insulin *versus* Se-insulin[C6U$^A$, C11U$^A$]. Fig. 10B presents a comparison of the unfolding transitions of WT human insulin and Se-insulin[C6U$^A$, C11U$^A$] as a function of the concentration of guanidine hydrochloride. The unfolding reactions were monitored at 25 °C by ellipticity at helix-sensitive wavelength 222 nm. Fig. 10C presents a comparison of CD spectra of insulin *lispro* (an engineered insulin monomer and active component of Humalog®; Eli Lilly) *versus* an analogue of insulin *lispro* containing paired substitutions [C6U$^A$, C11U$^A$]. Fig. 10D presents a comparison of the unfolding transitions of WT human insulin and Se-insulin[C6U$^A$, C11U$^A$] as a function of the concentration of guanidine hydrochloride; the unfolding reactions were monitored as in (B). Fig. 10E depicts the CD spectra of four selenium-containing [C6U$^A$, C11U$^A$] insulin analogues relative to their native counterparts: WT human insulin, insulin *lispro,* insulin *aspart* (Asp$^{B28}$-insulin), and insulin *glulisine* (Lys$^{B3}$, Glu$^{B29}$-insulin). Fig. 10F provides the respective guanidine unfolding transitions of the eight analogues shown in Fig. 10E; data were obtained under the same conditions employed in Fig. 10D.

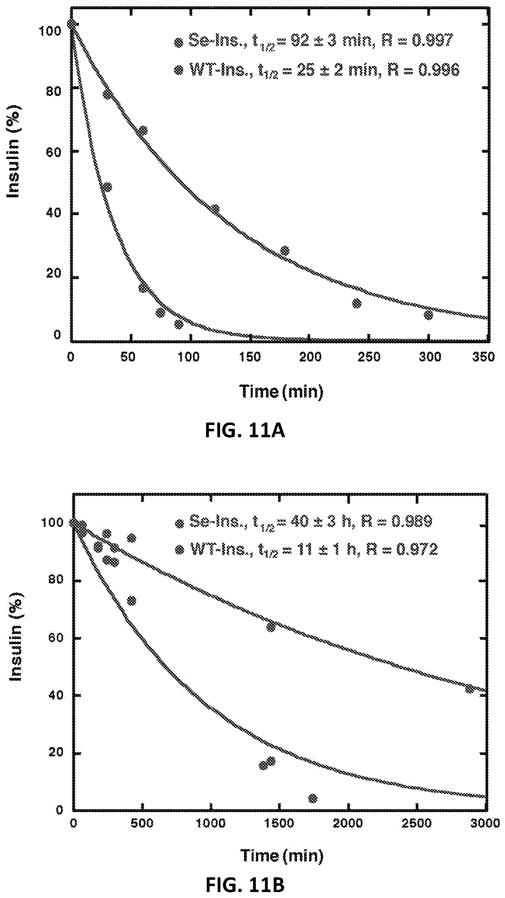

Figs. 11A-11C provide three distinct assays of protein stability. Fig 11A is a graph showing the time course of degradation of WT-insulin and Se-insulin[C6U[A], C11U[A]] by V8 protease. In this kinetic assay the seleno-analogue is roughly four times more stable than the WT-insulin, with half life $t_{1/2}$ = 92 $\pm$ 3 min *versus* 25 $\pm$ 2 min, respectively. Fig 11B is a graph showing the resistance to reductive unfolding of WT-insulin and Se-insulin[C6U[A], C11U[A]] by glutathione in its reduced form. Se-insulin[C6U[A], C11U[A]] was more resistant to reductive conditions with $t_{1/2}$ = 40 $\pm$ 3 h *versus* 11 $\pm$ 1 h for the WT-insulin. Standard deviations were within $\pm$5%. The data points were fit to a first-order decay function using KaleidaGraph to estimate rate constants, which were used in turn to calculate half lives ($t_{1/2}$). Fig 11C is a graph summarizing the respective kinetic features of insulin fibrillation for each analogue. Box plot of lag times prior to onset of fibrillations under accelerated conditions at 37 °C. The upper- and lower boundaries of boxes represent first and third quartiles, respectively. Upper and lower whiskers demarcate 1.5 times the interquartile range. Mean lag times are represented by gray horizontal lines, and median lag times by black horizontal lines. Lag times of individual replicates are represented by black diamonds; p values of each pair were < 0.05.

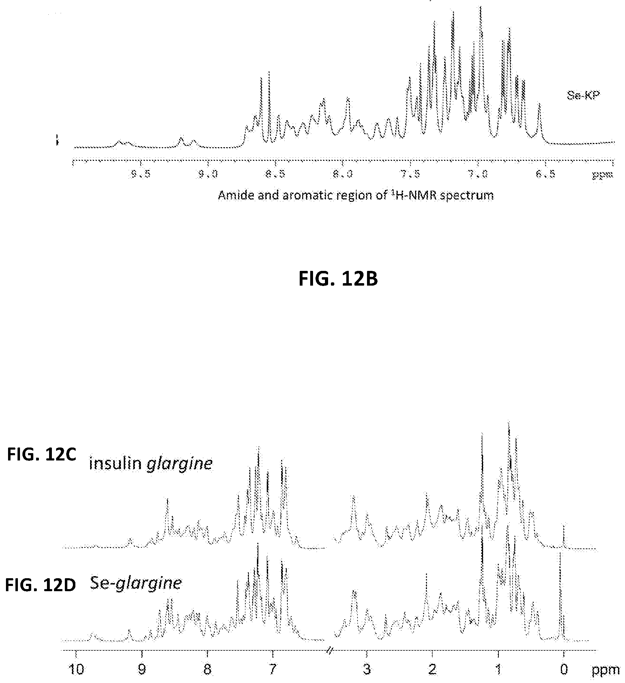

Fig. 12A-D provide $^1$H-NMR spectra of insulin analogs. Fig. 12A & 12B are one-dimensional (1D) $^1$H-NMR spectra of insulin *lispro* (Fig. 12A) *versus* its [C6U[A], C11U[A]] analogue (Fig. 12B) in 10 mM deuterio-acetic acid at pH 3.0 and 25 °C (90% $H_2O$ and 10% $D_2O$). An overall similarity was observed in chemical shifts and extent of chemical-shift dispersion. Sharpening of amide resonances was observed in the diselenide-stabilized analogue (inset; shaded box at top left), suggesting damping of conformational exchange on the millisecond time scale leading to resonance broadening in the parent analogue. Fig 12C & 12D provide corresponding 1D spectra of insulin *glargine* (Fig. 12C) *versus* its [C6U[A], C11U[A]] analogue (Fig. 12D). The latter spectrum exhibits more prominent amide resonances and upfield-shifted aliphatic resonances. Conditions were as in Fig. 12A and 12B. The magnetic field strength corresponded to a proton resonance frequency of 700 MHz.

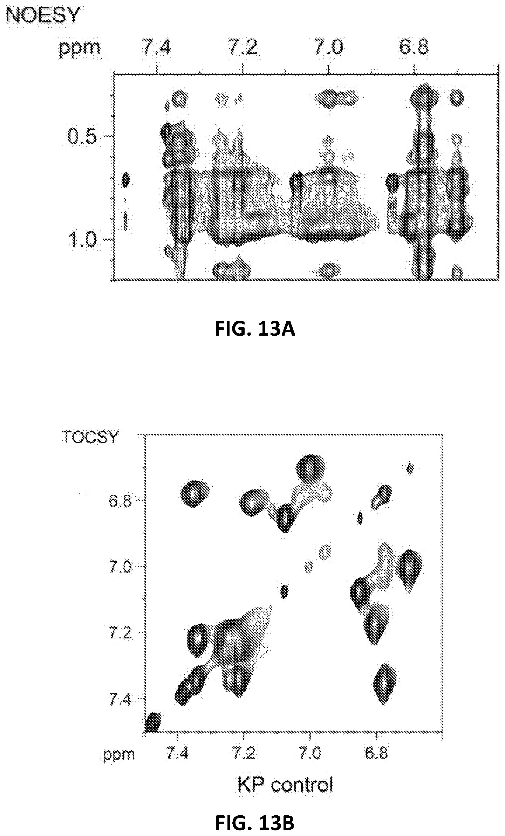

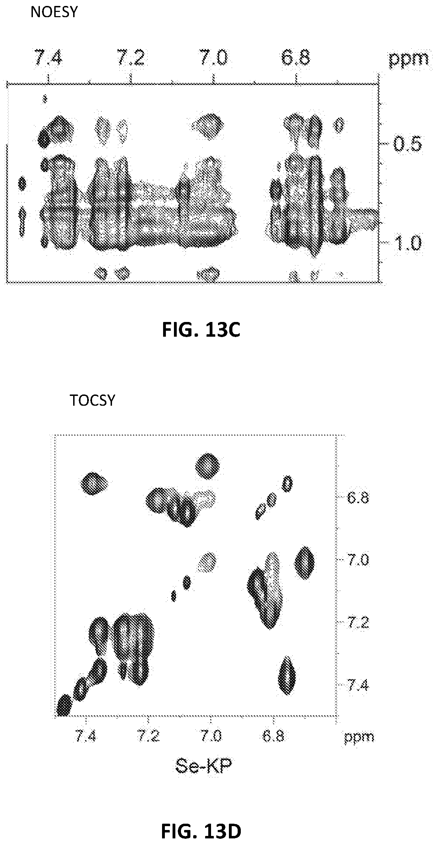

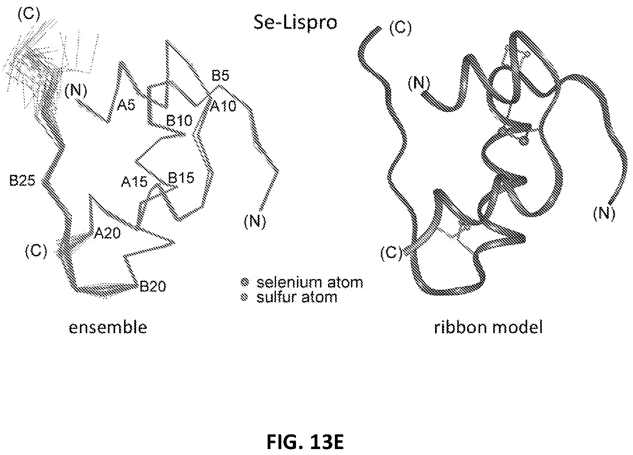

Figs. 13A-13E provide two-dimensional (2D) $^1$H-NMR spectra of insulin *lispro* (Figs. 13A and 13B) *versus* its [C6U[A], C11U[A]] analogue (Figs. 13C and 13D) in 10 mM deuterio-acetic acid at pH 3.0 and 25 °C. Figs. 13A and 13C provide respective 2D NOE cross peaks between aromatic protons (horizontal axis) and aliphatic protons (vertical axis; predominantly the methyl resonances of Ile, Leu and Val). Figs. 13B and 13D show the respective aromatic regions of the 2D TOCSY spectra. Spectra were obtained in 10 mM deuterio-acetic acid at pD 3.0 (direct meter reading) and 25 °C (99.9% $D_2O$) at 700 MHz. Fig. 13E provides the solution structure of the [C6U[A], C11U[A]] analogue of insulin *lispro*. An ensemble of structures is shown at left, and a representative ribbon model at right. The solution structure closely resembles that of insulin *lispro*. The NMR-derived structure of [C6U[A], C11U[A]]-modified insulin *lispro* was calculated on the basis of 866 NOEs as interproton distance restraints and 77 dihedral-angle restraints.

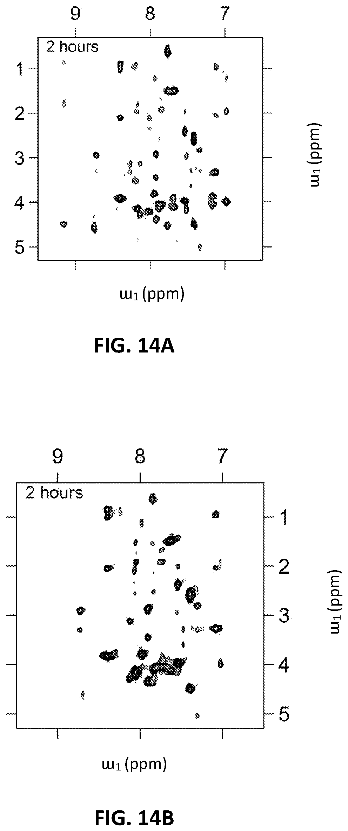

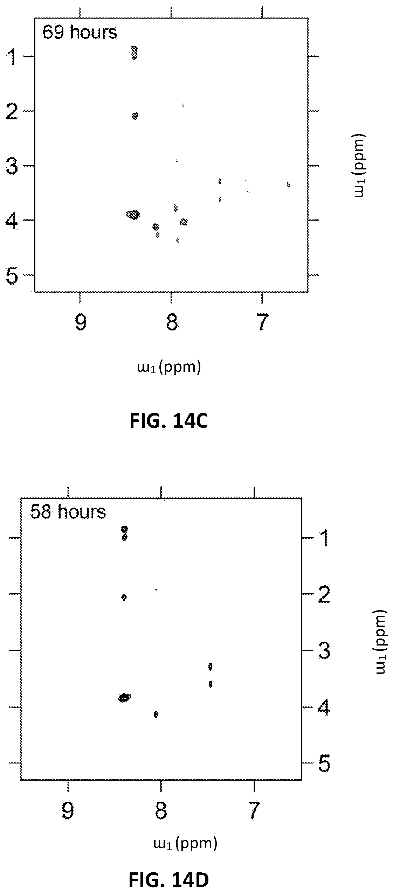

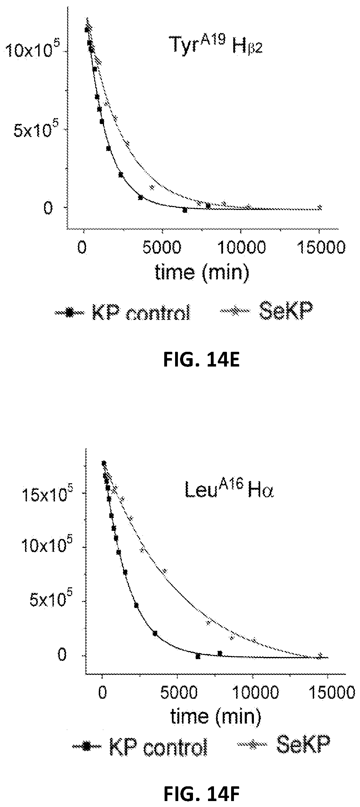

Figs. 14A-14F provide $^1$H-$^2$H amide-proton exchange studies of insulin *lispro versus* its [C6U[A], C11U[A]] analogue as a probe of thermodynamic stability. Figs. 14A & 14B provide 2D TOCSY spectra of [C6U[A], C11U[A]]-insulin *lispro* (Fig. 14A) and parent analogue (Fig. 14B) obtained two hours after dissolution in $D_2O$. Figs. 14C & 14D provide 2D TOCSY spectra of [C6U[A], C11U[A]]-insulin *lispro* (Fig. 14C; obtained 69 hours after dissolution in $D_2O$) and parent analogue (Fig. 14D; obtained 58 hours after dissolution in $D_2O$). Fig. 14E and 14F provides plots of the time course of amide-proton exchange for two (Try[A19]; Fig. 14E and Leu[A16]; Fig. 14F) whose exchange requires global unfolding. In each case exchange of insulin *lispro* (black) is more rapid than that of the corresponding amide proton in [C6U[A], C11U[A]]-insulin *lispro* (gray). This difference at pH 3.0 is smaller than is inferred at pH 7.4 from two-state modeling of guanidine unfolding transitions (see Figs. 10D & 10F above), but in the same general direction.

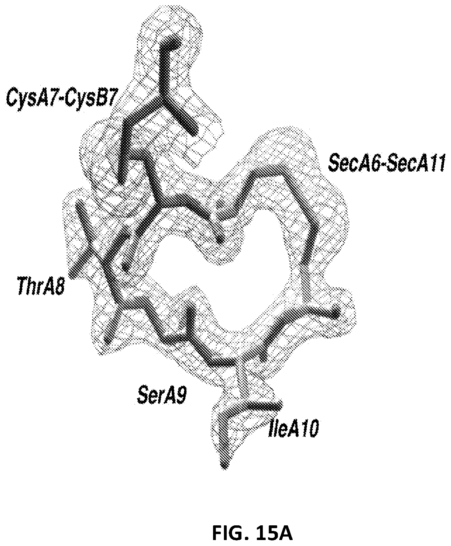

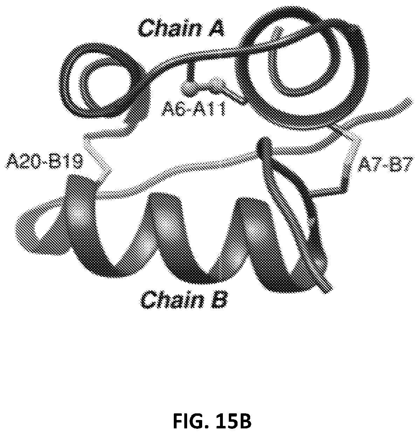

Figs. 15A & 15B depict structural representations of Se-insulin monomer. Fig. 15A depicts the X-ray crystal structure of Se-insulin[C6U[A], C11U[A]] and representative electron density. The electron density map corresponding to the region surrounding the A6-A11 diselenide bridge, demonstrating a very good fit of the model of this segment to the corresponding experimental density. Fig. 15B provides a representative structure of a Se-insulin monomer, displaying the diselenide bridge formed between Sec6 and Sec11 of chain A. The structure was extracted from an octameric assembly of Se-insulin protomers in the asymmetric unit of the crystal (not shown).

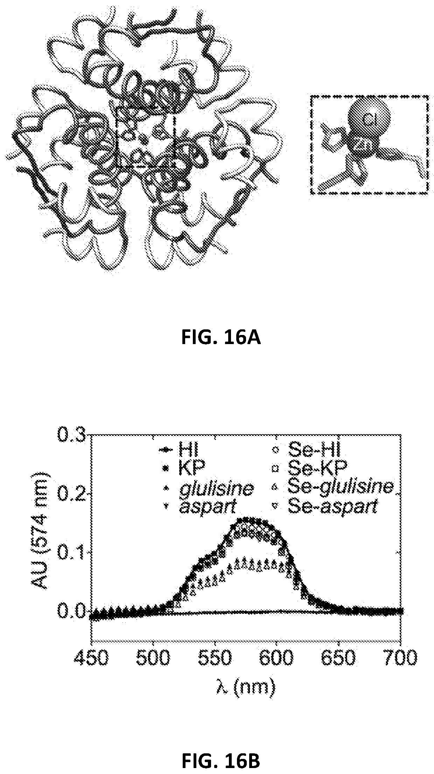

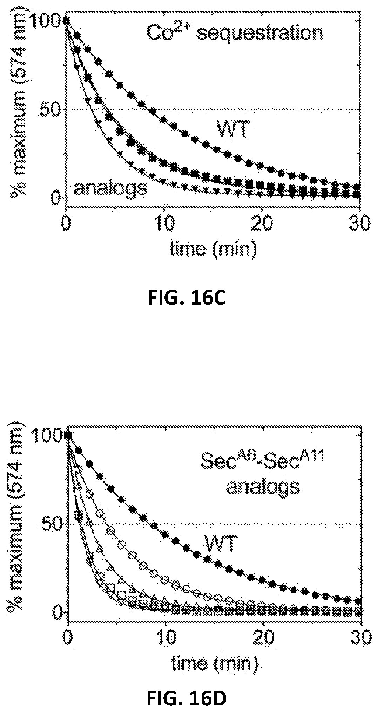

Figs. 16A-16D provides the visible absorption spectra of $Co^{2+}$-substituted insulin analogue hexamers and illustrates kinetic studies of their dissociation. Fig. 16A depicts the structure of an WT $R_6$ insulin hexamer. The tetrahedral $Zn^{2+}$-coordination site shown as ball-and-stick model in dotted box (insert): three His[B10] side chains and one chloride ion. Fig. 16B provides visible absorbance spectra of d-d bands in corresponding $Co^{2+}$ complex; the analogue codes are indicated. Fig. 16C presents hexamer dissociation curves as monitored at 574 nm after addition of excess EDTA; the analogue code is as in Fig. 16B. Fig. 16D presents the corresponding dissociation data pertaining to [C6U[A], C11U[A]]-insulin hexamers. The lifetime of the [C6U[A], C11U[A]]-insulin analogues were profoundly accelerated (compare solid black line for WT human insulin).

## DETAILED DESCRIPTION

**DEFINITIONS**

In describing and claiming the invention, the following terminology will be used in accordance with the definitions set forth below.

The term "about" as used herein means greater or lesser than the value or range of values stated by 10 percent, but is not intended to designate any value or range of values to only this broader definition. Each value or range of values preceded by the term "about" is also intended to encompass the embodiment of the stated absolute value or range of values.

As used herein the term "amino acid" encompasses any molecule containing both amino and carboxyl functional groups, wherein the amino and carboxylate groups are attached to the same carbon (the alpha carbon). The alpha carbon optionally may have one or two further organic substituents. For the purposes of the present disclosure designation of an amino acid (*e.g.,* by reference to the amino-acid single-letter code) without specifying its stereochemistry is intended to encompass either the L or D form of the amino acid, or a racemic mixture. However, in the instance where an amino acid is designated by its three-letter code and includes a superscript number, the D form of the amino acid is specified by inclusion of a lower case d before the three-letter code and superscript number (*e.g.*, dLys$^{-1}$), wherein the designation lacking the lower case d (*e.g.*, Lys$^{-1}$) is intended to specify the native L form of the amino acid. In this nomenclature, the inclusion of the superscript number designates the position of the amino acid in the sequence of the insulin analogue, wherein amino acids that are located within this sequence are designated by positive superscript numbers numbered consecutively from the N-terminus. Additional amino acids linked to the insulin analogue peptide either at the N-terminus or through a side chain are numbered starting with 0 and increasing in negative integer value as they are further removed from the insulin analogue sequence. Additional amino acids linked to the C-terminal residues of an A- or B chain are numbered as consequetive residues; for example, the C-terminal Arg-Arg extension of the 32-residue B chain in insulin *glargine* is designed Arg$^{B31}$-Arg$^{B32}$.

As used herein the term "non-coded amino acid" encompasses any amino acid that is not an L-isomer of any of the following 20 amino acids: Ala, Cys, Asp, Glu, Phe, Gly, His, Ile, Lys, Leu, Met, Asn, Pro, Gln, Arg, Ser, Thr, Val, Trp, Tyr.

A "bioactive polypeptide" refers to polypeptides that are capable of exerting a biological effect *in vitro* and/or *in vivo.*

As used herein a general reference to a peptide/polypeptide is intended to encompass peptides/polypeptides that have modified amino- and/or carboxy termini. For example, an amino-acid sequence designating the standard amino acids is intended to encompass standard amino acids at the N- and C terminus as well as modified amino acids, such as a corresponding C-terminal amino acid modified to comprise an amide group in place of the terminal carboxylic acid.

As used herein an "acylated" amino acid is an amino acid comprising an acyl group that is non-native to a naturally-occurring amino acid, regardless by the means by which it is produced. Exemplary methods of producing acylated amino acids and acylated peptides are known in the art; these include acylating an amino acid before inclusion in the peptide or chemical acylation of the peptide following its complete synthesis. In some embodiments the acyl group causes the peptide to have one or more of (i) a prolonged half-life in circulation, (ii) a delayed onset of action, (iii) an extended duration of action, (iv) an improved resistance to proteases and (v) increased or decreased potency at insulin receptor isoforms.

As used herein, an "alkylated" amino acid is an amino acid containing an alkyl group that is non-native to a naturally-occurring amino acid, regardless of the means by which it is produced. Exemplary methods of producing alkylated amino acids and alkylated peptides are known in the art; these nclude alkylating an amino acid before inclusion in the peptide or chemical alkylation of the peptide following its synthesis. Without being held to any particular theory, it is believed that alkylation of peptides will achieve similar, if not the same, effects as acylation of the peptides, *e.g.,* a prolonged half-life in circulation, a delayed onset of action, an extended duration of action, an improved resistance to proteases and increased or decreased potency.

As used herein, the term "pharmaceutically acceptable carrier" includes any of the standard pharmaceutical carriers, such as a phosphate-buffered saline (PBS) solution, water, emulsions such as an oil/water or water/oil emulsion, and various types of wetting agents. The term also encompasses any of the agents approved by a regulatory agency of the US Federal government or listed in the US Pharmacopeia for use in animals, including humans.

As used herein the term "pharmaceutically acceptable salt" encompasses salts of compounds that retain the biological activity of the parent compound and that are not biologically or otherwise undesirable. Many of the compounds disclosed herein are capable of forming acid and/or base salts by virtue of the presence of amino and/or carboxyl groups or groups similar thereto.

As used herein, the term "hydrophilic moiety" encompasses any compound that is readily water-soluble or readily absorbs water, and that are tolerated *in vivo* by mammalian species without toxic effects (*i.e.*, are biocompatible). Examples of hydrophilic moieties include polyethylene glycol (PEG), polylactic acid, polyglycolic acid, a polylactic-polyglycolic acid copolymer, polyvinyl alcohol, polyvinylpyrrolidone, polymethoxazoline, polyethyloxazoline, polyhydroxyethyl methacrylate, polyhydroxypropyl methacrylamide, polymethacrylamide, polydimethylacrylamide, and derivatised celluloses such as hydroxymethylcellulose or hydroxyethylcellulose and co-polymers thereof, as well as natural polymers including, for example, albumin, heparin and dextran.

As used herein, the term "treating" includes prophylaxis of the specific disorder or condition, or alleviation of the symptoms associated with a specific disorder or condition and/or preventing or eliminating said symptoms. For example, as used herein the term "treating diabetes" (or "treating DM") will refer in general to maintaining blood-glucose concentrations near normal levels and may include increasing or decreasing blood-glucose levels depending on a given situation.

As used herein, an "effective" amount or a "therapeutically effective amount" of an insulin analogue refers to a nontoxic but sufficient amount of an insulin analogue to provide the desired effect. For example one desired effect would be the prevention or treatment of hyperglycemia. The amount that is "effective" will vary from subject to subject, depending on the age and general condition of the individual, mode of administration, nutritional status and the like. Thus, it is not always possible to specify an exact "effective amount." However, an appropriate "effective" amount in any individual case may be determined by one of ordinary skill in the art using routine experimentation.

The term "parenteral" means not through the alimentary canal but by some other route such as intranasal, inhalation, subcutaneous (SQ), intramuscular, intraspinal, or intravenous (IV).

Throughout this application, all references to a particular amino-acid position by letter and number (*e.g.*, position A5) refer to the amino acid at that position of either the A chain (*e.g.,* position A5 with respect to SEQ ID NO: 2) or the B chain (*e.g.,* position B5 with respect to SEQ ID NO: 4) or the corresponding amino-acid position in any insulin analogues thereof. For example, a reference herein to "position B28" absent any further elaboration could mean residue Pro$^{B28}$ in WT insulin or the corresponding position 27 of a variant B chain in an insulin analogue in which the first amino acid of SEQ ID NO: 4 has been deleted (*des*-B1). Similarly, amino acids added to the N-terminus of the native B chain are numbered starting with B0, followed by numbers of increasing negative value (*e.g.,* B-1, B-2...) as amino acids are added to the N-terminus.

As used herein, the term "native human insulin peptide" is intended to designate the 51 amino-acid heteroduplex comprising the A chain of SEQ ID NO: 2 and the B chain of SEQ ID NO: 4, as well as single-chain insulin (SCI) analogues that comprise SEQ ID NOS: 2 and 4 (*i.e.*, as an A domain and a B domain). The term "insulin polypeptide" or "insulin peptide" as used herein, absent further descriptive language is intended to encompass the 51 amino-acid heteroduplex comprising the A chain of SEQ ID NO: 2 and the B chain of SEQ ID NO: 4; single-chain insulin analogues containing the native C domain of proinsulin, foreshortened C domains, novel connecting peptides, or non-peptidic linkers between the C terminus of the B chain and N-terminis of the A chain are herein collectively designated SCIs (including for example those disclosed in published international application WO96/34882 and US Patent No. 6,630,348. The class of SCIs thus contains homologous peptide hormones (*e.g.,* IGF1 and IGF2) and their variants that have activity at one or both of the insulin receptor isoforms. Such modified analogues include amino-acid modifications at one or more amino positions selected from A5, A8, A9, A10, A12, A14, A15, A17, A18, A21, B1, B2, B3, B4, B5, B9, B10, B13, B14, B17, B20, B21, B22, B23, B26, B27, B28, B29 and B30 or deletions of any or all of positions B1-4 and B26-30. Insulin polypeptides as defined herein can also be analogues derived from a naturally occurring insulin by insertion or substitution of a non-peptide moiety, *e.g.,* a retroinverso fragment, or incorporation of non-peptide bonds such as an azapeptide bond (CO substituted by NH) or pseudo-peptide bond (*e.g.*, NH substituted with $CH_2$) or an ester bond (*e.g.,* a depsipeptide, wherein one or more of the amide (-CONHR-) bonds are replaced by ester (COOR) bonds).

As used herein, the term "insulin A chain," absent further descriptive language is intended to encompass the 21 amino-acid sequence of SEQ ID NO: 2 as well as functional analogues and derivatives thereof known to those skilled in the art, including modification of the sequence of SEQ ID NO: 2 by one or more amino-acid substitutions at positions selected from A4, A5, A8, A9, A10, A12, A14, A15, A17, A18, A21.

As used herein, the term "insulin B chain," absent further descriptive language is intended to encompass the 30 amino-acid sequence of SEQ ID NO: 4, as well as modified functional analogues of the native B chain, including one or more amino-acid substitutions at positions selected from B1, B2, B3, B4, B5, B9, B10, B13, B14, B17, B20, B21, B22, B23, B25, B26, B27, B28, B29 and B30 or deletions of any or all of positions B 1-4 and B26-30.

As used herein, the term "derivative" is intended to encompass chemical modification to a compound (*e.g.,* an amino acid), including chemical modification *in vitro, e.g.,* by introducing a group in a side chain in one or more positions of a polypeptide, *e.g.,* a nitro group in a tyrosine residue or iodine in a tyrosine residue, or by conversion of a free carboxylic group to an ester group or to an amide group, or by converting an amino group to an amide by acylation, or by acylating a hydroxy group rendering an ester, or by alkylation of a primary amine rendering a secondary amine or linkage of a hydrophilic moiety to an amino-acid side chain. Other derivatives are obtained by oxidation or reduction of the side chains of the amino-acid residues in the polypeptide.

The term "identity" as used herein relates to the similarity between two or more sequences. Identity is measured by dividing the number of identical residues by the total number of residues and multiplying the product by 100 to achieve a percentage. Thus, two copies of exactly the same sequence have 100% identity, whereas two sequences that have amino-acid deletions, additions, or substitutions relative to one another have a lower degree of identity. Those skilled in the art will recognize that several computer programs, such as those that employ algorithms such as BLAST (Basic Local Alignment Search Tool, Altschul et al. (1993) J. Mol. Biol. 215:403-410), are available for determining sequence identity.

As used herein, an amino-acid "modification" refers to a substitution of an amino acid, or the derivation of an amino acid by the addition and/or removal of chemical groups to/from the amino acid, and includes substitution with any of the 20 amino acids commonly found in human proteins, as well as atypical or non-naturally occurring amino acids. Commercial sources of atypical amino acids include Sigma-Aldrich (Milwaukee, WI), ChemPep Inc. (Miami, FL), and Genzyme Pharmaceuticals (Cambridge, MA). Atypical amino acids may be purchased from commercial suppliers, synthesized *de novo,* or chemically modified or derivatized from naturally occurring amino acids.

As used herein, an amino-acid "substitution" refers to the replacement of one amino-acid residue by a different amino-acid residue.

As used herein, the term "conservative amino-acid substitution" is defined herein as exchanges within one of the following five groups:

I. Small aliphatic, nonpolar or slightly polar residues:
Ala, Ser, Thr, Pro, Gly;
II. Polar, negatively charged residues and their amides:
Asp, Asn, Glu, Gln, cysteic acid and homocysteic acid;
III. Polar, positively charged residues:
His, Arg, Lys; Ornithine (Orn)
IV. Large, aliphatic, nonpolar residues:
Met, Leu, Ile, Val, Cys, Norleucine (Nle), homocysteine
V. Large, aromatic residues:
Phe, Tyr, Trp, acetyl phenylalanine

As used herein, the general term "polyethylene-glycol chain" (or "PEG chain") encompasses mixtures of condensation polymers of ethylene oxide and water, in a branched or straight chain, represented by the general formula $H(OCH_2CH_2)_nOH$, wherein n is at least 2. "Polyethylene-glycol chain" (or "PEG chain") is used in combination with a numeric suffix to indicate the approximate average molecular weight thereof. For example, PEG-5,000 refers to polyethylene-glycol chain having a total molecular weight average of about 5,000 Daltons.

As used herein, the term "pegylated" and like terms include any compound that has been modified from its native state by linking a polyethylene-glycol chain to the compound. A "pegylated polypeptide" is a polypeptide that has a PEG chain covalently bound to the polypeptide.

As used herein, a "linker" is a bond, molecule or group of molecules that binds two separate entities to one another. Linkers may provide for optimal spacing of the two entities or may further supply a labile linkage that allows the two entities to be separated from each other. Labile linkages include photocleavable groups, acid-labile moieties, base-labile moieties and enzyme-cleavable groups.

As used herein, an "insulin dimer" is a complex comprising two insulin molecules (each containing an A and B chain) bound to each other through reversible non-covalent interactions, such as van der Waal interactions, electrostatic interactions and hydrogen bonds. Solutions of insulin in the absence of zinc ions at neutral pH and at protein concentrations greater than 1 $\mu$M typically exhibit an equilibrium distribution of monomeric insulin molecules, insulin dimers and higher-order oligomers. The term insulin dimer, when used absent any qualifying language, encompasses both insulin homodimers and insulin heterodimers. An insulin homodimer comprises two identical insulin polypeptides, whereas an insulin heterodimer comprises two insulin polypeptides that differ; an example of an insulin heterodimer would be provided by the association of a human insulin molecule with a bovine insulin molecule. As used herein, the term "covalent insulin dimer" designates two insulin molecules connected to each other by one or more a non-native covalent bonds; an example of such a bond would be an intermolecular disulfide bridge. Formation of covalent insulin dimers is known in the art as a mechanism of chemical degradation.

As used herein, the term "patient" without further designation is intended to encompass any warm-blooded vertebrate domesticated animal (including for example, but not limited to livestock, horses, cats, dogs and other pets) and humans.

The term "isolated" as used herein means having been removed from its natural environment. In some embodiments the analogue is made through recombinant methods wherein the analogue is isolated from the host cell, which typically may be a bacterial cell, yeast cell, inset cell or mammalian cell.

The term "purified" as used herein encompasses the isolation of a molecule or compound in a form that is substantially free of contaminants normally associated with the molecule or compound in a native or natural environment; in practice this means having been increased in purity as a result of being separated from other components of the original composition. The term "purified polypeptide" is used herein to describe a polypeptide which has been separated from other compounds including, but not limited to nucleic-acid molecules, lipids and carbohydrates.

ABBREVIATIONS:

Insulin analogues will be abbreviated as follows:

The insulin A- and B chains will be designated by a capital A for the A chain and a capital B for the B chain wherein a superscript 0 (*e.g.*, $A^0$ or $B^0$) will designate the base sequence is an insulin sequence (A chain: SEQ ID NO: 2, B chain SEQ ID NO: 4). Modifications that deviate from the native insulin are indicated in parenthesis following the designation of the A or B chain (*e.g.*, [$B^1$(H5,H10,Y16,L17) : $A^1$(H8,N18,N21)]) with the single-letter amino-acid abbreviation indicating the substitution and the number indicating the position of the substitution in the respective A or B chain, using native insulin numbering. A colon between the A and B chain indicates a two-chain insulin.

## EMBODIMENTS

The present disclosure is directed toward an insulin analogue that exhibits enhanced stability *in vitro* due to pairwise substitution of $Cys^{A6}$ and $Cys^{A11}$ by selenocysteine (three-letter code Sec; single-letter code U) as illustrated in Fig. 2C. The replacement of an internal disulfide bridge by an internal diselenide bridge at this position enhances the efficiency of core packing within the interior of the insulin molecule. Although not wishing to be limited by theory, this larger bridge between residues A6 and A11 damps conformational fluctuations that otherwise contribute to the susceptibility of WT human insulin to chemical and physical degradation-with special attention to the mechanism of fibrillation. It is another feature of the present disclosure that a diselenide bridge between residues A6 and A11 augments the thermodynamic stability of the insulin monomer and by so doing also protects the insulin dimer and zinc insulin hexamer, even if association constants are similar or reduced.

It is yet another feature of the present disclosure that $Sec^{A6}$ and $Sec^{A11}$ may be introduced pairwise into diverse analogues of human insulin, such as insulin *lispro,* insulin *aspart* and insulin *glulisine* (rapid-acting insulin analogue in current clinical use by patients with DM) and likewise confer favorable stabilization and protection from degradation. Introduction of a diselenide bridge between residues A6 and A11 thereby provides a general approach to extend the shelf-life of insulin analogue products. It is a further aspect of the present disclosure that the disclosed modification to the core of insulin does not significantly alter its receptor-binding surface, such that the pharmacologic, biological and therapeutic properties of the diselenide-stabilized derivative are similar to those of the parent insulin analogue.

In accordance with one embodiment an insulin peptide analogue is provided wherein a pairwise substitution of $Cys^{A6}$ and $Cys^{A11}$ in WT human insulin by $Sec^{A6}$ and $Sec^{A11}$ is made. It is another embodiment that these substitutions are introduced into a series of insulin analogues known in the art to exhibit more rapid absorption on SQ injection than does WT insulin; examples are provided by insulin *lispro* ($Pro^{B28}{\rightarrow}Lys$ and $Lys^{B29}{\rightarrow}Pro$; Eli Lilly), insulin *aspart* ($Pro^{B28}{\rightarrow}Asp$; Novo-Nordisk) and insulin *glulisine* ($Lys^{B29}{\rightarrow}Glu$ and $Asn^{B3}{\rightarrow}Lys$; Sanofi). Alternative embodiments may employ ornithine, diaminobutyric acid or diaminopropionic acid in place of lysine at positions B28 or B3. In yet other embodiments the parent insulin analogue is a long-acting hormone as known in the art; examples are provided by insulin *detemir* (Levemir®; Novo-Nordisk), insulin *degludec* (Tresiba®; Novo-Nordisk) and insulin *glargine* (Lantus® and Toujeo®; Sanofi). The present disclosure envisions that all these current insulin products can be favorably modified by the present disclosure to provide improved products, *i.e.,* with longer shelf life. The above set of embodiments can be further modified to include amino-acid substitutions known to occur naturally among vertebrate insulins, such as $Ala^{B30}$ instead of $Thr^{B30}$ (as is characteristic of porcine insulin and bovine insulin).

Furthermore, in view of the similarity between human and animal insulins, and use in the past of animal insulins in human patients with diabetes mellitus, it is also envisioned that other minor modifications in the sequence of insulin may be introduced, especially those substitutions considered "conservative." For example, additional substitutions of amino acids may be made within groups of amino acids with similar side chains, without departing from the present invention. These include the neutral hydrophobic amino acids: Alanine (Ala or A), Valine (Val or V), Leucine (Leu or L), Isoleucine (Ile or I), Proline (Pro or P), Tryptophan (Trp or W), Phenylalanine (Phe or F) and Methionine (Met or M). Likewise, the neutral polar amino acids may be substituted for each other within their group of Glycine (Gly or G), Serine (Ser or S), Threonine (Thr or T), Tyrosine (Tyr or Y), Cysteine (Cys or C), Glutamine (Glu or Q), and Asparagine (Asn or N). Acidic amino acids are Aspartic acid (Asp or D) and Glutamic acid (Glu or E). Introduction of basic amino-acid substitutions (including Lysine (Lys or K), Arginine (Arg or R) and Histidine (His or H)) are not preferred in order to maintain the enhanced net negative charge of this class of analogues. Unless noted otherwise or wherever obvious from the context, the amino acids noted herein should be considered to be *L*-amino acids. Standard amino acids may also be substituted by non-standard amino acids belonging to the same chemical class.

It is a general feature of the present disclosure that pairwise substitution of $Cys^{A6}$ and $Cys^{A11}$ by $Sec^{A6}$ and $Sec^{A11}$ enhances the speed and fidelity of insulin chain combination. The B chain in such reactions may be synthetic or biosynthetic in origin; its sequence may conform to that of WT insulin or contain amino-acid substitutions intended to modify the pharmacologic, biophysical or biological properties of the final insulin analogue. It is yet another general feature of the present disclosure that pairwise substitution of $Cys^{A6}$ and $Cys^{A11}$ by $Sec^{A6}$ and $Sec^{A11}$ likewise enhances the speed and fidelity of oxidative folding of single-chain insulin analogues, as exemplified by Des-Di (a 49-residue polypeptide containing substitution $Pro^{B28} \rightarrow Lys$ and a peptide bond between $Lys^{B28}$ and $Gly^{A1}$ with deletion of residues B29 and B30). A diselenide bridge at positions A6 and A11 is also anticipated to enhance the folding of longer single-chain insulin precursor polypeptides, including proinsulin itself (86 residues).

In one embodiment the presently disclosed insulin analogues have been reduced to practice as illustrated by a molecular embodiment in the context of insulin *lispro*, insulin *aspart*, insulin *glulisine* and insulin *glargine* The first three examples (prandial analogues in current clinical use; respectively known as Humalog® [Eli Lilly and Co.], Novolog® [Novo-Nordisk] and Apidra® [Sanofi])) demonstrate the utility of paired selenocysteine substitutions at positions A6 and A11 within a prototypical rapid-acting insulin in current clinical use, including in pumps. The fourth analogue is the active ingredient of basal insulin analogue formulation in current clinical use (Lantus® and Toujeo®; Sanofi)..

As background, an analogue of WT human insulin containing pairwise substitution of $Cys^{A6}$ and $Cys^{A11}$ by $Sec^{A6}$ and $Sec^{A11}$ was prepared by insulin chain combination. This protocol employs a synthetic A chain and a WT B chain, obtained either by oxidative sulfitolysis of biosynthetic human insulin (as employed herein) or chemical peptide synthesis. The general protocol for solid-phase synthesis is as described (Merrifield et al., 1982. Biochemistry 21: 5020-5031).

As described in the Examples, the [C6U$^A$, C11U$^A$] analogue of WT human insulin exhibited native-like structure and function with enhanced stability (relative to WT insulin). Examples are provided based on diselenide-based protection of three prandial insulin analogues in current clinical use: insulin *lispro*, insulin *aspart* and insulin *glulisine*.

The present disclosure provides the basis for improved products to treat DM by means of a pharmaceutical formulation intended for SQ injection. In still another example, the insulin analogue is administered by an external or implantable insulin pump. A pharmaceutical composition may comprise such insulin analogues and may optionally include zinc. In one embodiment zinc ions may not be needed to stabilize a pharmaceutical formulation due to the intrinsic stability conferred by the presently disclosed diselenide bridge at positions A6 and A11. Zinc ions may nonetheless be included in such a composition at a level of a molar ratio of between 2.2 and 3.0 per hexamer of the insulin analogue; higher concentrations of zinc ions may be present in acidic formulations of pI-shifted insulin analogues such as insulin *glargine.* In such formulations the concentration of the insulin analogue would typically be between about 0.1 and about 3 mM; concentrations up to 3 mM may be used in the reservoir of an insulin pump. Modifications of meal-time insulin analogues may be formulated as described for (a) "regular" formulations of Humulin® (Eli Lilly and Co.), Humalog® (Eli Lilly and Co.), Novalin® (Novo-Nordisk), and Novolog® (Novo-Nordisk) and other rapid-acting insulin formulations currently approved for human use, (b) "NPH" formulations of the above and other insulin analogues, and (c) mixtures of such formulations. (c) Yet another class of pharmaceutical formulations encompass long-acting insulin products as exemplified by U-100 and U-300 strengths of insulin *glargine* under acidic conditions (Sanofi) and acylated insulin analogues such as insulin *detemir* and insulin *degludec* at neutral pH (Novo-Nordisk).

Excipients may include glycerol, glycine, other buffers and salts, and anti-microbial preservatives such as phenol and *meta-cresol;* the latter preservatives are known to enhance the stability of the insulin hexamer. Such a pharmaceutical composition may be used to treat a patient having diabetes mellitus or other medical condition by administering a physiologically effective amount of the composition to the patient.

Based upon the foregoing disclosure, it should now be apparent that [Sec$^{A6}$, Sec$^{A11}$]-substituted insulin analogues will carry out the objects set forth hereinabove. Namely, these insulin analogues exhibit enhanced thermodynamic stability, resistance to fibrillation with maintenance of potency in reducing blood-glucose levels at a level similar to that of the parent insulin analogue.

In one embodiment an insulin receptor agonist analogue is provided wherein the analogue comprises an insulin A chain and an insulin B chain wherein

said A chain comprises a sequence of
$GIVX_4X_5X_6CX_8X_9X_{10}X_{11}X_{12}LX_{14}X_{15}LX_{17}X_{18}YCX_{21}-R_{53}$ (SEQ ID NO: 10), and
said B chain comprises a sequence
$R_{62}-X_{25}LCGX_{29}X_{30}LVX_{33}X_{34}LYLVCGX_{41}X_{42}GFX_{45}$ (SEQ ID NO: 11), wherein
$X_4$ is glutamic acid or aspartic acid;
$X_5$ is glutamine or glutamic acid;
$X_6$ is selenocysteine;
$X_8$ is histidine, threonine, lysine, arginine, tryptophan or phenylalanine;
$X_9$ is serine, arginine, lysine, ornithine or alanine;
$X_{10}$ is isoleucine or serine;
$X_{11}$ is selenocysteine;
$X_{12}$ is serine or aspartic acid;
$X_{14}$ is tyrosine, arginine, lysine, ornithine or alanine;
$X_{15}$ is glutamine, glutamic acid, arginine, alanine, lysine, ornithine or leucine;
$X_{17}$ is glutamic acid, aspartic acid, asparagine, lysine, ornithine or glutamine;

$X_{18}$ is methionine, asparagine, glutamine, aspartic acid, glutamic acid or threonine;

$X_{21}$ is selected from the group consisting of alanine, glycine, serine, valine, threonine, isoleucine, leucine, glutamine, glutamic acid, asparagine, aspartic acid, histidine, tryptophan, tyrosine, and methionine;

$X_{25}$ is histidine or threonine;

$X_{29}$ is selected from the group consisting of alanine, glycine and serine;

$X_{30}$ is selected from the group consisting of histidine, aspartic acid, glutamic acid, homocysteic acid and cysteic acid;

$X_{33}$ is selected from the group consisting of aspartic acid and glutamic acid;

$X_{34}$ is selected from the group consisting of alanine and threonine;

$X_{41}$ is selected from the group consisting of glutamic acid, aspartic acid and asparagine;

$X_{42}$ is selected from the group consisting of alanine, ornithine, lysine and arginine;

$X_{45}$ is tyrosine or phenylalanine;

$R_{62}$ is selected from the group consisting of AYRPSE (SEQ ID NO: 12), FVNQ (SEQ ID NO: 13), PGPE (SEQ ID NO: 14), a tripeptide glycine-proline-glutamic acid, a tripeptide valine-asparagine-glutamine, a dipeptide proline-glutamic acid, a dipeptide asparagine-glutamine, glutamine, glutamic acid and an N-terminal amine; and

$R_{53}$ is COOH or $CONH_2$, wherein a diselenide bridge is formed between the side chains of residues A6 and A11. In one embodiment $X_4$ is glutamic acid or aspartic acid; $X_5$ is glutamine or glutamic acid; $X_6$ is selenocysteine; $X_8$ is histidine, threonine or phenylalanine; $X_9$ is serine or arginine; $X_{10}$ is isoleucine or serine; $X_{11}$ is selenocysteine; $X_{12}$ is serine or aspartic acid; $X_{14}$ is tyrosine, arginine or alanine; $X_{15}$ is glutamine, arginine, or leucine; $X_{17}$ is glutamic acid or aspartic acid; $X_{18}$ is methionine, asparagine or threonine; $X_{21}$ is selected from the group consisting of alanine, glycine and asparagine; $X_{25}$ is histidine or threonine; $X_{29}$ is selected from the group consisting of alanine, glycine and serine; $X_{30}$ is selected from the group consisting of histidine, aspartic acid and glutamic acid; $X_{33}$ is selected from the group consisting of aspartic acid and glutamic acid; $X_{34}$ is selected from the group consisting of alanine and threonine; $X_{41}$ is selected from the group consisting of glutamic acid and aspartic acid; $X_{42}$ is arginine; and $X_{45}$ is tyrosine or phenylalanine.

In one embodiment an insulin analogue is provided wherein the insulin A chain comprises the sequence GIVEQX$_6$CX$_8$SIX$_{11}$SLYQLENYCX$_{21}$-R$_{53}$ (SEQ ID NO: 15) and the insulin B chain comprises the sequence FVX$_{23}$QX$_{25}$LCGSHLVEALYLVCGERGFFYTX$_{48}$X$_{49}$X$_{50}$ (SEQ ID NO: 16), wherein

$X_6$ is seleno-cysteine;

$X_8$ is histidine, threonine, lysine, arginine, phenylalanine or tryptophan;

$X_{11}$ is seleno-cysteine; and

$X_{21}$ is selected from the group consisting of alanine, glycine, serine, threonine, glutamine, asparagine, and aspartic acid;

$X_{23}$ is Asn or Lys:

$X_{25}$ is histidine or threonine;

$X_{48}$ is proline, lysine or aspartic acid;

$X_{49}$ is proline, lysine or glycine;

$X_{50}$ is threonine, alanine or serine; and

$R_{53}$ is COOH or $CONH_2$.

In one embodiment an insulin analogue is provided wherein the A chain comprises the sequence GIVEQX$_6$CX$_8$X$_9$IX$_{11}$SLYQLENYCX$_{21}$-R$_{53}$ (SEQ ID NO: 17) and said B chain comprises the sequence FVX$_{23}$QX$_{25}$LCGX$_{29}$X$_{30}$LVX$_{33}$X$_{34}$LYLVCGX$_{41}$X$_{42}$GFX$_{45}$ (SEQ ID NO: 18), wherein

$X_6$ is selenocysteine;

$X_8$ is histidine or threonine;

$X_9$ is serine, lysine, or alanine;

$X_{11}$ is selenocysteine;

$X_{21}$ is alanine, glycine or asparagine;

$X_{23}$ is is asparagine or lysine;

$X_{25}$ is histidine or threonine;

$X_{29}$ is selected from the group consisting of alanine, glycine and serine;

$X_{30}$ is selected from the group consisting of histidine, aspartic acid, glutamic acid, homocysteic acid and cysteic acid;

$X_{33}$ is selected from the group consisting of aspartic acid and glutamic acid;

$X_{34}$ is selected from the group consisting of alanine and threonine;

$X_{41}$ is selected from the group consisting of glutamic acid, aspartic acid and asparagine;

$X_{42}$ is selected from the group consisting of alanine, ornithine, lysine and arginine;
$X_{45}$ is tyrosine or phenylalanine;
$R_{53}$ is COOH or $CONH_2$.

In a further embodiment the A chain comprises the sequence

GIVEQX₆CX₈X₉IX₁₁SLYQLENYCX₂₁-R₅₃ (SEQ ID NO: 17) and said B chain comprises the sequence FVNQHLCGSHLVEALYLVCGERGFFY (SEQ ID NO: 50), or
FVKQHLCGSHLVEALYLVCGERGFFY (SEQ ID NO: 51, wherein

$X_6$ is selenocysteine;
$X_8$ is histidine or threonine;
$X_9$ is serine, lysine, or alanine;
$X_{11}$ is selenocysteine;
$X_{21}$ is alanine, glycine or asparagine; and
$R_{53}$ is COOH or $CONH_2$.

In a further embodiment the A chain comprises the sequence GIVEQX₆CX₈X₉IX₁₁SLYQLENYCX₂₁-R₅₃ (SEQ ID NO: 17) and said B chain comprises a sequence selected from the group consisting of

FVNQHLCGSHLVEALYLVCGERGFFYTPKT (SEQ ID NO: 4),
FVNQHLCGSHLVEALYLVCGERGFFYTPKT (SEQ ID NO: 4),
FVNQHLCGSHLVEALYLVCGERGFFYTDKT (SEQ ID NO: 5),
FVNQHLCGSHLVEALYLVCGERGFFYTKPT (SEQ ID NO: 6),
FVKQHLCGSHLVEALYLVCGERGFFYTPET (SEQ ID NO: 7),
FVNQHLCGSHLVEALYLVCGERGFFYTPKTRR (SEQ ID NO: 8), and
FVNQHLCGSHLVEALYLVCGERGFFYTPX₂₀X₃₀ (SEQ ID NO: 9), wherein

$X_6$ is selenocysteine;
$X_8$ is histidine or threonine;
$X_9$ is serine, lysine, or alanine;
$X_{11}$ is selenocysteine;
$X_{21}$ is alanine, glycine or asparagine;
$X_{29}$ is an acyl derivative of lysine or glutamic-acid-bridged acyl derivative of Lysine
$X_{30}$ is Thr, Ala, Ser or absent; and
$R_{53}$ is COOH or $CONH_2$.

In another embodiment the A chain comprises the sequence GIVEQX₆CX₈SIX₁₁SLYQLENYCX₂₁-R₅₃ (SEQ ID NO: 15) and said B chain comprises a sequence selected from the group consisting of

FVNQHLCGSHLVEALYLVCGERGFFYTPKT (SEQ ID NO: 4),
FVNQHLCGSHLVEALYLVCGERGFFYTDKT (SEQ ID NO: 5),
FVNQHLCGSHLVEALYLVCGERGFFYTKPT (SEQ ID NO: 6), and
FVKQHLCGSHLVEALYLVCGERGFFYTPET (SEQ ID NO: 7),
wherein

$X_6$ is selenocysteine;
$X_8$ is histidine or threonine;
$X_{11}$ is selenocysteine;
$X_{21}$ is alanine, glycine or asparagine; and
$R_{53}$ is COOH or $CONH_2$.

In one embodiment an insulin analogue is provided wherein the A chain comprises a sequence GIVEQX₆CTSIX₁₁SLYQLENYCN (SEQ ID NO: 30) and said B chain comprises a sequence selected from the group consisting of FVNQHLCGSHLVEALYLVCGERGFFYTPKT (SEQ ID NO: 4), FVNQHLCGSHLVEALYLVCGERGFFYTDKT (SEQ ID NO: 5),

FVNQHLCGSHLVEALYLVCGERGFFYTKPT (SEQ ID NO: 6),

FVKQHLCGSHLVEALYLVCGERGFFYTPET (SEQ ID NO: 7),
FVNQHLCGSHLVEALYLVCGERGFFYTPKTRR (SEQ ID NO: 8), and
FVNQHLCGSHLVEALYLVCGERGFFYTP$X_{20}X_{30}$ (SEQ ID NO: 9), wherein

$X_6$ is selenocysteine;
$X_{11}$ is selenocysteine;
$X_{29}$ is an acyl derivative of lysine or glutamic-acid-bridged acyl derivative of lysine, and
$X_{30}$ is Thr, Ala, Ser or absent.

In one embodiment an insulin analogue is provided wherein the A chain comprises a sequence GIVEQX$_6$CT-SIX$_{11}$SLYQLENYCN (SEQ ID NO: 30) and the B chain comprises a sequence FVNQHLCGSHLVEALYLVCGERGFFYTPKT (SEQ ID NO: 4), wherein $X_6$ is selenocysteine and $X_{11}$ is selenocysteine.

In accordance with one embodiment the insulin analogue is a two-chain analogue wherein the A and B chains are joined to one another by two disulfide (S-S) bridges whereas the A chain contains a diselenide (Se-Se) bridge.

In one embodiment an insulin analogue is provided wherein the A chain comprises the sequence GIVEQX$_6$CT-SIX$_{11}$SLYQLENYCN-R$_{53}$ (SEQ ID NO: 30) and the B chain sequence comprises the sequence FVKQX$_{25}$LCGSHLVEA-LYLVCGERGFF-R$_{63}$ (SEQ ID NO: 21), or FVNQX$_{25}$LCGSHLVEALYLVCGERGFF-R$_{63}$ (SEQ ID NO: 20), wherein

$X_6$ is selenocysteine;
$X_{11}$ is selenocysteine;
$X_{25}$ is selected from the group consisting of histidine and threonine;
$R_{53}$ is COOH or CONH$_2$; and
$R_{63}$ is selected from the group consisting of YTX$_{28}$KT (SEQ ID NO: 22), YTKPT (SEQ ID NO: 23), YTX$_{28}$K (SEQ ID NO: 24), YTKP (SEQ ID NO: 25), YTPK (SEQ ID NO: 26), YTX$_{28}$, YT, Y and a bond, wherein $X_{28}$ is proline, lysine, aspartic acid or glutamic acid. In a further embodiment the A chain comprises the sequence GIVEQX$_6$CTSIX$_{11}$SLYQ-LENYCN-R$_{53}$ (SEQ ID NO: 30) as above and the B chain sequence comprises the sequence

FVKQX$_{25}$LCGSHLVEALYLVCGERGFFYTEKT (SEQ ID NO: 28),
FVNQX$_{25}$LCGSHLVEALYLVCGERGFFYTDKT (SEQ ID NO: 27),
FVNQX$_{25}$LCGSHLVEALYLVCGERGFFYTKPT (SEQ ID NO: 29) or
FVNQX$_{25}$LCGSHLVEALYLVCGERGFFYTPKT (SEQ ID NO: 30) wherein
$X_{25}$ is selected from the group consisting of histidine and threonine, optionally wherein $X_{25}$ is histidine.

Covalently linkage of a hydrophilic moiety to the insulin analogues disclosed herein provides analogues having slower onset, extended duration and exhibit a basal profile of activity. In one embodiment, the insulin polypeptides disclosed herein are further modified to comprise a hydrophilic moiety covalently linked to the side chain of an amino acid at a position selected from the group consisting of A9, A14 and A15 of the A chain or at the N-terminal alpha amine of the B chain (*e.g.,* at position B1 for an insulin-based B chain) or at the side chain of an amino acid at position B1, B2, B10, B22, B28 or B29 of the B chain. In exemplary embodiments, this hydrophilic moiety is covalently linked to a Lys, Cys, Orn, homocysteine, or acetyl-phenylalanine residue at any of these positions.

Exemplary hydrophilic moieties include polyethylene glycol (PEG), for example, of a molecular weight of about 1,000 Daltons to about 40,000 Daltons, or about 20,000 Daltons to about 40,000 Daltons. Additional suitable hydrophilic moieties include, polypropylene glycol, polyoxyethylated polyols (*e.g.,* POG), polyoxyethylated sorbitol, polyoxyethylated glucose, polyoxyethylated glycerol (POG), polyoxyalkylenes, polyethylene glycol propionaldehyde, copolymers of ethylene glycol/propylene glycol, monomethoxy-polyethylene glycol, mono-(C1-C10) alkoxy- or aryloxy-polyethylene glycol, carboxymethylcellulose, polyacetals, polyvinyl alcohol (PVA), polyvinyl pyrrolidone, poly-1, 3-dioxolane, poly-1,3,6-trioxane, ethylene/maleic anhydride copolymer, poly (beta-amino acids) (either homopolymers or random copolymers), poly(n-vinyl pyrrolidone)polyethylene glycol, propropylene glycol homopolymers (PPG) and other polyakylene oxides, polypropylene oxide/ethylene oxide copolymers, colonic acids or other polysaccharide polymers, Ficoll or dextran and mixtures thereof.

The hydrophilic moiety, *e.g.*, polyethylene glycol chain in accordance with some embodiments has a molecular weight selected from the range of about 500 to about 40,000 Daltons. In one embodiment the hydrophilic moiety, *e.g.,* PEG, has a molecular weight selected from the range of about 500 to about 5,000 Daltons, or about 1,000 to about 5,000 Daltons. In another embodiment the hydrophilic moiety, *e.g.,* PEG, has a molecular weight of about 10,000 to about 20,000 Daltons. In yet other exemplary embodiment the hydrophilic moiety, *e.g.,* PEG, has a molecular weight of about 20,000 to about 40,000 Daltons. In one embodiment the hydrophilic moiety, *e.g.,* PEG, has a molecular weight of about 20,000 Daltons. In one embodiment an insulin polypeptide is provided wherein one or more amino acids of the analogue are pegylated, and the combined molecular weight of the covalently linked PEG chains is about 20,000 Daltons.

In one embodiment dextrans are used as the hydrophilic moiety. Dextrans are polysaccharide polymers of glucose subunits, predominantly linked by α1-6 linkages. Dextran is available in many molecular weight ranges, *e.g.,* about 1 kD to about 100 kD, or from about 5, 10, 15 or 20 kD to about 20, 30, 40, 50, 60, 70, 80 or 90 kD.

Linear or branched polymers are contemplated. Resulting preparations of conjugates may be essentially monodisperse or polydisperse, and may have about 0.5, 0.7, 1, 1.2, 1.5 or 2 polymer moieties per peptide.

In one embodiment the hydrophilic moiety is a polyethylene glycol (PEG) chain, optionally linked to the side chain of an amino acid at a position selected from the group consisting of A9, A14 and A15 of the A chain, positions B1, B2, B10, B22, B28 or B29 of the B chain, at the N-terminal alpha amine of the B chain.

In some embodiments, the insulin analogue is modified to comprise an acyl group. The acyl group can be covalently linked directly to an amino acid of the insulin polypeptide, or indirectly to an amino acid of the insulin polypeptide via a spacer, wherein the spacer is positioned between the amino acid of the insulin polypeptide and the acyl group. The insulin polypeptide may be acylated at the same amino-acid position where a hydrophilic moiety is linked, or at a different amino-acid position. For example, acylation may occur at any position including any amino acid of the A or B chains provided that the activity exhibited by the non-acylated insulin polypeptide is retained upon acylation. Nonlimiting examples include acylation at positions A14 and A15 of the A chain, or positions B10, B22, B28 or B29 of the B chain.

In one specific aspect of the disclosure, the insulin polypeptide (or derivative or conjugate thereof) is modified to comprise an acyl group by direct acylation of an amine, hydroxyl, or thiol of a side chain of an amino acid of the insulin polypeptide. In some embodiments, the insulin polypeptide is directly acylated through the side chain amine, hydroxyl, or thiol of an amino acid. In some embodiments, acylation is at position B28 or B29 (according to the amino-acid numbering of the native insulin A and B chain sequences). In this regard, an insulin polypeptide can be provided that has been modified by one or more amino acid substitutions in the A or B chain sequence, including for example at positions A14, A15, B1, B2, B10, B22, B28 or B29 (according to the amino-acid numbering of the native insulin A and B chain sequences) or at any position of the linking moiety with an amino acid comprising a side chain amine, hydroxyl, or thiol. In some specific embodiments of the disclosure, the direct acylation of the insulin polypeptide occurs through the side chain amine, hydroxyl, or thiol of the amino acid at position B28 or B29 (according to the amino acid numbering of the native insulin A and B chain sequences).

In accordance with one embodiment, the acylated insulin polypeptides comprise a spacer between the peptide and the acyl group. In some embodiments, the insulin polypeptide is covalently bound to the spacer, which is covalently bound to the acyl group. In some exemplary embodiments, the insulin polypeptide is modified to comprise an acyl group by acylation of an amine, hydroxyl, or thiol of a spacer, which spacer is attached to a side chain of an amino acid at position B28 or B29 (according to the amino-acid numbering of the A or B chain of native insulin), or at any position of the spacer moiety. The amino acid of the insulin polypeptide to which the spacer is attached can be any amino acid comprising a moiety which permits linkage to the spacer. For example, an amino acid comprising a side chain -NH$_2$, -OH, or -COOH (*e.g.,* Lys, Orn, Ser, Asp or Glu) is suitable.

In accordance with certain embodiments the bifunctional spacer can be a synthetic or naturally occurring amino acid comprising an amino acid backbone that is 3 to 10 atoms in length (*e.g.,* 6-amino hexanoic acid, 5-aminovaleric acid, 7-aminoheptanoic acid, and 8-aminooctanoic acid). Alternatively, the spacer can be a dipeptide or tripeptide spacer having a peptide backbone that is 3 to 10 atoms (*e.g.,* 6 to 10 atoms) in length. Each amino acid of the dipeptide or tripeptide spacer attached to the insulin polypeptide can be independently selected from the group consisting of: naturally-occurring and/or non-naturally occurring amino acids, including, for example, any of the D or L isomers of the naturally-occurring amino acids.

The insulin polypeptide can be modified to comprise an acyl group by acylation of a long-chain alkane. In specific aspects, the long-chain alkane comprises an amine, hydroxyl, or thiol group (*e.g.*, octadecylamine, tetradecanol, and hexadecanethiol) which reacts with a carboxyl group, or activated form thereof, of the insulin polypeptide. The carboxyl group, or activated form thereof, of the insulin polypeptide can be part of a side chain of an amino acid (*e.g.,* glutamic acid, aspartic acid) of the insulin polypeptide or can be part of the peptide backbone.

With regard to these aspects of the disclosure, in which a long-chain alkane is acylated by the peptide the insulin polypeptide or the spacer, the long-chain alkane may be of any size and can comprise any length of carbon chain. The long-chain alkane can be linear or branched. In certain aspects, the long-chain alkane is a C$_4$ to C$_{30}$ alkane. For example, the long-chain alkane can be any of a C$_4$ alkane, C$_6$ alkane, C$_8$ alkane, C$_{10}$ alkane, C$_{12}$ alkane, C$_{14}$ alkane, C$_{16}$ alkane, C$_{18}$ alkane, C$_{20}$ alkane, C$_{22}$ alkane, C$_{24}$ alkane, C$_{26}$ alkane, C$_{28}$ alkane, or a C$_{30}$ alkane. In some embodiments, the long-chain alkane comprises a C$_8$ to C$_{20}$ alkane, *e.g.,* a C$_{14}$ alkane, C$_{16}$ alkane, or a C$_{18}$ alkane.

In accordance with one embodiment a pharmaceutical composition is provided comprising any of the novel insulin dimers disclosed herein, preferably at a purity level of at least 90%, 91%, 92%, 93%, 94%, 95%, 96%, 97%, 98% or 99%, and a pharmaceutically acceptable diluent, carrier or excipient. Such compositions may contain an insulin dimer as disclosed herein at a concentration of at least 0.5 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml or higher. In one embodiment the pharmaceutical compositions comprise aqueous solutions that are sterilized and optionally stored contained within various package containers. In other embodiments the pharmaceutical compositions comprise a lyophilized powder. The pharmaceutical compositions can be further packaged as part of a kit that includes a disposable device for administering the composition to a patient. The containers or kits may be labeled for storage at ambient room temperature or at refrigerated temperature.

The disclosed insulin analogues are believed to be suitable for any use that has previously been described for insulin peptides. Accordingly, the insulin analogues disclosed herein can be used to treat hyperglycemia, or treat other metabolic diseases that result from high blood-glucose levels. Accordingly, the present disclosure encompasses pharmaceutical compositions comprising an insulin analogues as disclosed herein and a pharmaceutically acceptable carrier for use in treating a patient suffering from high blood-glucose levels. In accordance with one embodiment the patient to be treated using an insulin analogue disclosed herein is a domesticated animal, and in another embodiment the patient to be treated is a human. We also envision that pairwise substitution of $Cys^{A6}$ and $Cys^{A11}$ may be useful in any member of the insulin superfamily (in which these cysteines are invariant) to enhance the efficiency, speed and fidelity of oxidative folding and to stabilize the folded state once obtained; this superfamily includes vertebrate proinsulins, vertebrate insulin-like growth factors (IGF-1 and IGF-2), vertebrate relaxins, and invertebrate insulin-like proteins and analogues thereof.

One method of treating hyperglycemia in accordance with the present disclosure comprises the steps of administering the presently disclosed insulin analogues to a patient using any standard route of administration, including parenterally, such as intravenously, intraperitoneally, subcutaneously or intramuscularly, intrathecally, transdermally, rectally, orally, nasally or by inhalation. In one embodiment the composition is administered subcutaneously or intramuscularly. In one embodiment, the composition is administered parenterally and the insulin polypeptide, or prodrug derivative thereof, is prepackaged in a syringe.

The insulin analogues disclosed herein may be administered alone or in combination with other anti-diabetic agents or anti-obesity agents. Anti-diabetic agents known in the art or under investigation include insulin, leptin, Peptide YY (PYY), Pancreatic Peptide (PP), fibroblast growth factor 21 (FGF21), Y2Y4 receptor agonists, sulfonylureas, such as tolbutamide (Orinase), acetohexamide (DYMELOR®), tolazamide (Tolinase), chlorpropamide (DIABINESE®), glipizide (GLUCOTROL®), glyburide (DIABETA®, MICRONASE®, GLYNASE®), glimepiride (AMARYL®), or gliclazide (Diamicron); meglitinides, such as repaglinide (PRANDIN®) or nateglinide (STARLIX®); biguanides such as metformin (GLUCOPHAGE®) or phenformin; thiazolidinediones such as rosiglitazone (AVANDIA®), pioglitazone (ACTOS®), or troglitazone (Rezulin), or other PPARγ inhibitors; alpha glucosidase inhibitors that inhibit carbohydrate digestion, such as miglitol (GLYSET®), acarbose (PRECOSE®/GLUCOBAY®); exenatide (BYETTA®) or pramlintide; dipeptidyl peptidase-4 (DPP-4) inhibitors such as vildagliptin or sitagliptin; SGLT (sodium-dependent glucose transporter 1) inhibitors; glucokinase activators (GKA); glucagon receptor antagonists (GRA); or FBPase (fructose 1,6-bisphosphatase) inhibitors..

Anti-obesity agents known in the art or under investigation include appetite suppressants, including phenethylamine type stimulants, phentermine (optionally with fenfluramine or dexfenfluramine), diethylpropion (TENUATE®), phendimetrazine (PRELU-2®, BONTRIL®), benzphetamine (DIDREX®), sibutramine (MERIDIA®, REDUCTIL®); rimonabant (ACOMPLIA®), other cannabinoid receptor antagonists; oxyntomodulin; fluoxetine hydrochloride (PROZAC®); Qnexa (topiramate and phentermine), EXCALIA® (bupropion and zonisamide) or CONTRAVE® (bupropion and naltrexone); or lipase inhibitors, similar to XENICAL (Orlistat) or Cetilistat (also known as ATL-962), or GT 389-255.

Pharmaceutical compositions comprising the insulin analogues disclosed herein can be formulated and administered to patients using standard pharmaceutically acceptable carriers and routes of administration known to those skilled in the art. Accordingly, the present disclosure also encompasses pharmaceutical compositions comprising one or more of the insulin analogues disclosed herein (or prodrug derivative thereof), or a pharmaceutically acceptable salt thereof, in combination with a pharmaceutically acceptable carrier. In one embodiment the pharmaceutical composition comprises a 1 mg/ml concentration of the insulin analogue at a pH of about 4.0 to about 7.0 in a phosphate buffer system. The pharmaceutical compositions may comprise the insulin analogue as the sole pharmaceutically active component, or the insulin analogue can be combined with one or more additional active agents.

In one embodiment a kit is provided with a device for administering an insulin analogue composition to a patient. The kit may further include a variety of containers, *e.g.*, vials, tubes, bottles, and the like. Preferably, the kits will also include instructions for use. In another embodiment the kit comprises a syringe and a needle, and in one embodiment the insulin analogue composition is prepackaged within the syringe.

The compounds of this disclosure may be prepared by standard synthetic methods, recombinant DNA techniques, or any other methods of preparing peptides and fusion proteins. Although certain non-natural amino acids cannot be expressed by standard recombinant DNA techniques, techniques for their preparation are known in the art. Compounds of this disclosure that encompass non-peptide portions may be synthesized by standard organic-chemistry reactions, in addition to standard peptide-chemistry reactions when applicable.

In accordance with embodiment 1 an insulin analogue comprising an insulin A chain and an insulin B chain is provided wherein said A chain comprises a sequence of $GIVX_4X_5X_6CX_8X_9X_{10}X_{11}X_{12}LX_{14}X_{15}LX_{17}X_{18}YCX_{21}$-$R_{53}$ (SEQ ID NO: 10), and said B chain comprises a sequence $R_{62}$-$X_{25}LCGX_{29}X_{30}LVX_{33}X_{34}LYLVCGX_{41}X_{42}GFX_{45}$ (SEQ ID NO: 11), wherein $X_4$ is glutamic acid or aspartic acid; $X_5$ is glutamine or glutamic acid; $X_6$ is selenocysteine; $X_8$ is histidine,

threonine, lysine, arginine, tryptophan or phenylalanine; $X_9$ is serine, arginine, lysine, ornithine or alanine; $X_{10}$ is isoleucine or serine; $X_{11}$ is selenocysteine; $X_{12}$ is serine or aspartic acid; $X_{14}$ is tyrosine, arginine, lysine, ornithine or alanine; $X_{15}$ is glutamine, glutamic acid, arginine, alanine, lysine, ornithine or leucine; $X_{17}$ is glutamic acid, aspartic acid, asparagine, lysine, ornithine or glutamine; $X_{18}$ is methionine, asparagine, glutamine, aspartic acid, glutamic acid or threonine; $X_{21}$ is selected from the group consisting of alanine, glycine, serine, valine, threonine, isoleucine, leucine, glutamine, glutamic acid, asparagine, aspartic acid, histidine, tryptophan, tyrosine, and methionine; $X_{25}$ is histidine or threonine; $X_{29}$ is selected from the group consisting of alanine, glycine and serine; $X_{30}$ is selected from the group consisting of histidine, aspartic acid, glutamic acid, homocysteic acid and cysteic acid; $X_{33}$ is selected from the group consisting of aspartic acid and glutamic acid; $X_{34}$ is selected from the group consisting of alanine and threonine; $X_{41}$ is selected from the group consisting of glutamic acid, aspartic acid and asparagine; $X_{42}$ is selected from the group consisting of alanine, ornithine, lysine and arginine; $X_{45}$ is tyrosine or phenylalanine; $R_{62}$ is selected from the group consisting of AYRPSE (SEQ ID NO: 12), FVNQ (SEQ ID NO: 13), PGPE (SEQ ID NO: 14), a tripeptide glycine-proline-glutamic acid, a tripeptide valine-asparagine-glutamine, a dipeptide proline-glutamic acid, a dipeptide asparagine-glutamine, glutamine, glutamic acid and an N-terminal amine; and $R_{53}$ is COOH or CONH$_2$, wherein a diselenide bridge is formed between the side chains of residues A6 and A11.

In embodiment 2 an insulin analogue of embodiment 1 is provided wherein $X_4$ is glutamic acid or aspartic acid; $X_5$ is glutamine or glutamic acid; $X_6$ is selenocysteine; $X_8$ is histidine, threonine or phenylalanine; $X_9$ is serine or arginine; $X_{10}$ is isoleucine or serine; $X_{11}$ is selenocysteine; $X_{12}$ is serine or aspartic acid; $X_{14}$ is tyrosine, arginine or alanine; $X_{15}$ is glutamine, arginine, or leucine; $X_{17}$ is glutamic acid or aspartic acid; $X_{18}$ is methionine, asparagine or threonine; $X_{21}$ is selected from the group consisting of alanine, glycine and asparagine; $X_{25}$ is histidine or threonine; $X_{29}$ is selected from the group consisting of alanine, glycine and serine; $X_{30}$ is selected from the group consisting of histidine, aspartic acid and glutamic acid; $X_{33}$ is selected from the group consisting of aspartic acid and glutamic acid; $X_{34}$ is selected from the group consisting of alanine and threonine; $X_{41}$ is selected from the group consisting of glutamic acid and aspartic acid; $X_{42}$ is arginine; and $X_{45}$ is tyrosine or phenylalanine.

In embodiment 3 an insulin analogue any one of embodiments 1-2 is provided wherein said A chain comprises the sequence GIVEQX$_6$CX$_8$SIX$_{11}$SLYQLENYCX$_{21}$-R$_{53}$ (SEQ ID NO: 15), wherein $X_6$ is seleno-cysteine; $X_8$ is histidine, threonine, lysine, arginine, or tryptophan; $X_{11}$ is seleno-cysteine; and $X_{21}$ is selected from the group consisting of alanine, glycine, serine, threonine, glutamine, asparagine, and aspartic acid.

In embodiment 4 an insulin analogue any one of embodiments 1-3 is provided wherein said B chain comprises the sequence FVX$_{23}$QX$_{25}$LCGSHLVEALYLVCGERGFFYTX$_{48}$X X$_{50}$ (SEQ ID NO: 16), wherein
$X_{23}$ is Asn or Lys:

$X_{25}$ is histidine or threonine;
$X_{48}$ is proline, lysine or aspartic acid;
$X_{49}$ is proline, lysine or glycine;
$X_{50}$ is threonine, alanine or serine; and
$R_{53}$ is COOH or CONH$_2$.

In embodiment 5 an insulin analogue any one of embodiments 1-4 is provided wherein said A chain comprises the sequence GIVEQX$_6$CX$_8$X$_9$IX$_{11}$SLYQLENYCX$_{21}$-R$_{53}$ (SEQ ID NO: 17, and said B chain comprises the sequence FVX$_{23}$QX$_{25}$LCGX$_{29}$X$_{30}$LVX$_{33}$X$_{34}$LYLVCGX$_{41}$X$_{42}$GFX$_{45}$ (SEQ ID NO: 18), wherein $X_6$ is selenocysteine; $X_8$ is histidine or threonine; $X_9$ is serine, lysine, or alanine; $X_{11}$ is selenocysteine; $X_{21}$ is alanine, glycine or asparagine; $X_{23}$ is asparagine or lysine;
$X_{25}$ is histidine or threonine; $X_{29}$ is selected from the group consisting of alanine, glycine and serine; $X_{30}$ is selected from the group consisting of histidine, aspartic acid, glutamic acid, homocysteic acid and cysteic acid; $X_{33}$ is selected from the group consisting of aspartic acid and glutamic acid; $X_{34}$ is selected from the group consisting of alanine and threonine; $X_{41}$ is selected from the group consisting of glutamic acid, aspartic acid and asparagine; $X_{42}$ is selected from the group consisting of alanine, ornithine, lysine and arginine; $X_{45}$ is tyrosine or phenylalanine; and $R_{53}$ is COOH or CONH$_2$.

In embodiment 6 an insulin analogue any one of embodiments 1-3 or 5 is provided wherein the B chain sequence comprises the sequence

FVKQX$_{25}$LCGSHLVEALYLVCGERGFF-R$_{63}$ (SEQ ID NO: 21), or
FVNQX$_{25}$LCGSHLVEALYLVCGERGFF-R$_{63}$ (SEQ ID NO: 20), wherein
$X_{25}$ is selected from the group consisting of histidine and threonine; and
$R_{63}$ is selected from the group consisting of YTX$_{28}$KT (SEQ ID NO: 22), YTKPT (SEQ ID NO: 23), YTX$_{28}$K (SEQ ID NO: 24), YTKP (SEQ ID NO: 25), YTPK (SEQ ID NO: 26), YTX$_{28}$, YT, Y and a bond, wherein $X_{28}$ is proline, lysine, aspartic acid or glutamic acid.

In embodiment 7 an insulin analogue any one of embodiments 1-6 is provided wherein the B chain sequence comprises the sequence

$\quad$ FVKQX$_{25}$LCGSHLVEALYLVCGERGFFYTEKT (SEQ ID NO: 28),
$\quad$ FVNQX$_{25}$LCGSHLVEALYLVCGERGFFYTDKT (SEQ ID NO: 27),
$\quad$ FVNQX$_{25}$LCGSHLVEALYLVCGERGFFYTKPT (SEQ ID NO: 29) or
$\quad$ FVNQX$_{25}$LCGSHLVEALYLVCGERGFFYTPKT (SEQ ID NO: 30) wherein
$\quad$ X$_{25}$ is selected from the group consisting of histidine and threonine.

In embodiment 8 an insulin analogue any one of embodiments 1-7 is provided wherein X$_{25}$ is histidine.

In embodiment 9 an insulin analogue any one of embodiments 1-8 is provided wherein the A chain is an amino-acid sequence consisting of GIVEQX$_6$CTSIX$_{11}$SLYQLENYCN-R$_{53}$ (SEQ ID NO: 30) and the B chain comprises the sequence
FVNQHLCGSHLVEALYLVCGERGFFYTPKT (SEQ ID NO: 4), wherein

$\quad$ X$_6$ is selenocysteine;
$\quad$ X$_{11}$ is selenocysteine and
$\quad$ R$_{53}$ is COOH or CONH$_2$.

In embodiment 10 an insulin analogue any one of embodiments 1-9 is provided wherein said A chain comprises the sequence GIVEQX$_6$CTSIX$_{11}$SLYQLENYCN (SEQ ID NO: 30) and said B chain comprises the sequence FVNQHLCGSHLVEALYLVCGERGFFYTPKT (SEQ ID NO: 4), wherein X$_6$ is selenocysteine and X$_{11}$ is selenocysteine.

In embodiment 11 an insulin analogue any one of embodiments 1-10 is provided wherein said A chain comprises the sequence GIVEQX$_6$CTSIX$_{11}$SLYQLENYCN-R$_{53}$ (SEQ ID NO: 30) and said B chain comprises the sequence

$\quad$ FVKQX$_{25}$LCGSHLVEALYLVCGERGFFYTEKT (SEQ ID NO: 28) or
$\quad$ FVNQX$_{25}$LCGSHLVEALYLVCGERGFFYTKPT (SEQ ID NO: 29), wherein

$\quad$ X$_{25}$ is histidine; and
$\quad$ R$_{53}$ is COOH or CONH$_2$.

In embodiment 12 an insulin analogue any one of embodiments 1-11 is provided wherein a hydrophilic moiety is covalently linked at one or more positions corresponding to A14, A15, B0, B1, B10, B22, B28, B29, optionally wherein the hydrophilic moiety is a polyethylene glycol.

In embodiment 13 an insulin analogue any one of embodiments 1-12 is provided, further comprising an acyl group or alkyl group covalently linked to an amino-acid side chain of said insulin analog, optionally wherein said acyl group or alkyl group is covalently linked to one or more positions selected from A14, A15, B0, B1, B10, B22, B28, B29 of the insulin peptide.

In embodiment 14 two or more insulin analogues any one of embodiments 1-13 are covalently linked to one another to form a dimer or multimer.

In embodiment 15 a pharmaceutical composition is provided comprising an insulin analogue any one of embodiments 1-14, or pharmaceutically acceptable salt thereof, and a pharmaceutically acceptable carrier.

In embodiment 16 a method of treating diabetes is provided, said method comprising administering an effective amount of a pharmaceutical composition of embodiment 15.

In embodiment 17, a pharmaceutical composition is provided comprising an insulin analogue of any one of embodiments 1-14, or pharmaceutically acceptable salt thereof and an excipient selected from the group consisting of triphosphate ions, trisulphate ions, and chelating agents, including (but not limited to) ethylene-diamine-tetra-acetic acid (EDTA) and ethylene glycol-bis(β-aminoethyl ether)-N,N,N',N'-tetraacetic acid (EGTA) and a pharmaceutically acceptable carrier.

**EXAMPLE 1**

Synthesis of [C6U, C11U]-Substituted Human Insulin

In brief, the 21-residue peptide GIVEQX$_6$CTSIX$_{11}$SLYQLENYCN-R$_{53}$ (SEQ ID NO: 31), wherein X$_6$ and X$_{11}$ are each Se (denoted Chain A[C6U, C11U]) was prepared using an automated peptide synthesizer (CS136XT, CS Bio Inc. CA) on a 0.25 mmol scale using Fmoc-Asn(Trt)-TentaGel®R PHB resin (loading of 0.17 mmol/g). Fmoc-amino acids (2 mmol in 5 mL DMF) activated with HCTU (2 mmol in 5 mL DMF) and DIEA (4 mmol in 5 mL DMF) for 5 min and allowed to couple for 25 min, with constant shaking. Fmoc-deprotection was carried out with 20% piperidine in DMF ($2 \times 5$ min). Fmoc-Sec(Mob)-OH was manually coupled for two hours using DIC/OxymaPure activation method (2.9 equiv/3.0 equiv respectively). The peptide-resin was then washed with DMF, DCM and dried under vacuum. The dried peptide-resin was cleaved in the presence of 2 equivalents of DTNP, using a

TFA:water:thioanisole:triisopropylsilane:ethanedithiol (92.5:1.5:1.5:1.5:1.5) cocktail for 4 h.

The cleavage mixture was filtered and TFA was evaporated with $N_2$ bubbling to minimum volume, to which 8-fold volume of cold ether was added dropwise. The precipitated crude peptide was centrifuged (5000 rpm, 10 min), ether was removed and the crude peptide dissolved in ACN-water (1:1) containing 0.1% TFA and was further diluted to ~25% ACN with water and lyophilized, giving 208 mg of crude material. The crude peptide were dissolved in 25% ACN in water containing 0.1% TFA and purified by multiple injections of 100 mg each using prep RP-HPLC (XSelect C18 column, 5 $\mu$m, $30 \times 250$ mm) using a gradient of 25%-50% B over 50 min; this step provided 49 mg of chain A[C6U, C11U] analogue (8% yield, based on resin loading). The product was characterized by UPLC using ACQUITY UPLC XSelect C18 column (3.5 $\mu$m, 130 Å, $4.6 \times 150$ mm), and a gradient of 5% B over 2 min then 5-70% B over 20 min and ESI-MS.

Analytical reverse-phase HPLC (rp-HPLC) was performed using a Waters UPLC H-Class or Waters Alliance HPLC with 220 nm UV detection using a XSelect C18 column (3.5 $\mu$m, 130 Å, $4.6 \times 150$ mm) or XBridge BEH300 C4 (3.5 $\mu$m, 130 Å, $4.6 \times 150$ mm). Preparative and semi-preparative rp-HPLC was performed on a Waters LCQ150 system using a XBridge C8 column (5 $\mu$m, $10 \times 150$ mm) or XSelect C18 column (5 $\mu$m, 130 Å, $30 \times 250$ mm). Linear gradients of ACN (with 0.1 % TFA, eluent B) in water (with 0.1 % TFA, eluent A) were used for all systems to elute bound peptides. The flow rates were 1 mL/min (analytical), 3.35 mL/min (Semi-preparative), and 20 mL/min (preparative). ESI-MS was performed on LCQ Fleet Ion Trap mass spectrometer (Thermo Scientific). Peptide masses were calculated from the experimental mass to charge (m/z) ratios from the observed multiply charged species of a peptide. The HR-MS were recorded on a Q-ExactivePlus Orbitrap mass spectrometer (Thermo Scientific) with a ESI source and 140'000 FWHM, in a method with AGC target set to 1E6, and scan range was 400-2800 m/z. Deconvolution of the raw experimental MS data was performed with the help of MagTran v1.03 software.

A solution of chain A[C6U, C11U] analogue (3.5 mg) was dissolved in a glycine buffer (0.1 M, pH 10.6) and mixed with a solution of sulfitolized chain B (4.85 mg). The final concentration of each chain was 1.2 mM in the same buffer. To initiate chain combination, DTT was added in quantity stoichiometric to the concentration of S-sulfonate groups (2.4 mM in this case). The reaction was carried out at 4 °C with open sample reaction to permit air oxidation. To control the reaction progress, small aliquots (10 $\mu$L) were taken from the reaction solution, quenched by addition of water containing 0.1% TFA (15 $\mu$L) and analyzed by injecting 20 $\mu$L of the sample onto the analytical rp-HPLC (XSelect C4 column, 3.5 $\mu$m, 130 Å, $4.6 \times 150$ mm); the HPLC protocol employed a gradient of 5% eluent B in eluent A for 1 min then 5-70% eluent B over 21 min, with detection at 220 nm. At the end of the recombination reaction, the Se-insulin[C6U$^A$, C11U$^A$] was isolated through a XBridge BEH300 analytical C4 column (3.5 $\mu$m, 130 Å, $4.6 \times 150$ mm) and lyophilized to yield 31% (2.6 mg) based on the amounts of the chains used.

**Table 1.** Summary of [C6U, C11U] Insulin Analogues (Example 2)

| parent analogue | [C6U. C11U]-modified analogue |
|---|---|
| insulin *lispro* | [Sec$^{A6}$, Sec$^{A11}$, Lys$^{B28}$, Pro$^{B29}$]-insulin |
| insulin *glulisine* | [Sec$^{A6}$, Sec$^{A11}$, Lys$^{B3}$, Glu$^{B29}$]-insulin |
| insulin *aspart* | [Sec$^{A6}$, Sec$^{A11}$, Asp$^{B28}$]-insulin |
| insulin *glargine* | [Sec$^{A6}$, Sec$^{A11}$, Gly$^{A21}$, Arg$^{B31}$, Arg$^{B32}$]-insulin |
| insulin *lispro analogue 1* | [Sec$^{A6}$, Sec$^{A11}$, His$^{A8}$, Lys$^{B28}$, Pro$^{B29}$]-insulin |
| insulin *lispro analogue 2* | [Sec$^{A6}$, Sec$^{A11}$, Glu$^{A14}$, Lys$^{B28}$, Pro$^{B29}$]-insulin |
| insulin *lispro analogue 3* | Sec$^{A6}$, Sec$^{A11}$, His$^{A8}$, Glu$^{A14}$, Lys$^{B28}$, Pro$^{B29}$]-insulin |
| insulin *lispro analogue 4* | [Sec$^{A6}$, Sec$^{A11}$, Asp$^{B10}$, Lys$^{B28}$, Pro$^{B29}$]-insulin |

## Example 2

[C6U, C11U] Insulin Analogues

Eight insulin analogues containing [C6U, C11U] (and so containing a diselenide bridge between positions A6 and A11) were prepared to exemplify the compatibility and utility of this bridge with standard amino-acid substitutions known in the art to alter the PK/PD properties of insulin or to augment its stability. These analogues are listed in Table 1. The first four represent [C6U, C11U]-reinforced versions of insulin analogues in current clinical use for the treatment of DM (insulins *lispro, glulisine, aspart* and *glargine*); the remaining four were prepared to investigate whether the gain in stability conferred by the A6-A11 diselenide bridge would be additive to mechanisms of stabilization known in the art to be conferred by standard amino-acid substitutions as exemplified by His$^{A8}$ Glu$^{A14}$ and Asp$^{B10}$. Each of these eight insulin analogues was mono-component as evaluated by analytical reversal-phase HPLC and exhibited its predicted mass as evaluated by mass spectrometry. Native-like folding was in each case verified by far ultra-violet circular dichroism (CD).

An analogue of insulin *lispro* containing pairwise substitution of Cys$^{A6}$ and Cys$^{A11}$ by Sec$^{A6}$ and Sec$^{A11}$ was prepared essentially as described in Example 1, except that the WT B chain (obtained from commercial insulin) was replaced by a synthetic analogue containing the paired substitutions Pro$^{B28}$→Lys and Lys$^{B29}$→Pro. Technical differences pertained to instrumentation. LC-MS analyses were performed on LCQ Advantage Ion Trap Mass Spectrometer System coupled with Agilent 1100 Series HPLC system. Analytical rp- HPLC was performed using a Waters 1525 Binary HPLC system. All chromatographic separations were performed on a C8 Proto (4.6x250 mm) 300 Å, 5 μm, Higgins Analytical Inc. column, using 25-50% Solvent B in Solvent A over 35 minutes (solvent A = 0.1% TFA in water, solvent B = 0.1% TFA in acetonitrile) at a flow rate of 1.0 mL/min with detection by UV absorption at 215 nm. Preparative rp-HPLC employed the Waters 2545 Quaternary pumping system equipped with FlexInject.

As described in detail below, the [C6U$^A$, C11U$^A$] analogue of insulin *lispro* exhibited native-like structure and function with enhanced stability (relative to insulin *lispro*).

The second approach was designed to exploit a 48-residue single-chain insulin (SCI) precursor, prepared by an automated solid-phase peptide synthesizer (Tribute 2-Channel peptide synthesizer using 6Cl-HOBt/DIC mediated coupling protocols). The synthetic protocol was as described by DiMarchi and colleagues (Zykov, A.N., et al. (2014) ACS Chem. Biol. 9, 683-91). Residues 1-28 of SCI precursor corresponded to insulin B-chain residues B1-B28, except that residue 28 was Lys instead of Pro; this Lysine (unique in the SCI sequence) provided a specific cleavage site for Endo-Lys C. Residues 29-48 of the SCI precursor corresponded to A-chain residues A1-21; the critical Sec$^{A6}$ and Sec$^{A11}$ substitutions thus were placed at peptide residues 34 and 39 in the SCI precursor. The reduced polypeptide underwent oxidative folding to form the A6-A11 diselende bond and cystines A7-B7 and B20-A19 essentially as described by Zykov, A.N., et al. (2014).

The folded SCI analogue was digested with trypsin to liberate a large fragment, designated des-octapeptide[B23-B30]-insulin-A[C6U, C11U]; DOI-A[C6U, C11U]. This fragment thus contained a truncated B-chain fragment (residues B1-B22) and intact A-chain analogue (A[C6U$^A$, C11U$^A$] as above). The two native disulfide bridges and novel A6-A11 diselenide bridge were contained within this fragment. An octapeptide of sequence Gly-Phe-Phe-Tyr-Thr-Lys-Pro-Thr was in parallel prepared by automated peptide synthesis. The desired analogue of insulin *lispro* containing a A6-A11 diselenide bridge will then be obtained by trypsin-mediated semi-synthesis as described (Kubiac, T. and Cowburn, D. (1986) Int. J. Pept. Protein Res. 27, 514-21). In brief, the formation of a peptide bond between Arg$^{B22}$ and the synthetic octapeptide will be mediated by trypsin (in a mixed solvent system containing 1,4-butanediol and dimethylacetamide). Insulin analogues will be purified by preparative C4 rp-HPLC (Higgins Analytical Inc, Proto 300 C4 10 μM, 250 x 20 mm), and their purity assessed by analytical rp-C4 HPLC (Higgins C4 5μM, 250 x 4.6 mm). Molecular masses of purified intermediates and analogues were verified using mass spectrometry as above.

An analogue of insulin *glulisine* likewise containing pairwise substitution of Cys$^{A6}$ and Cys$^{A11}$ by Sec$^{A6}$ and Sec$^{A11}$ was prepared by chain combination essentially as described above in Example 2, except that the WT B chain (obtained from commercial insulin) was replaced by a synthetic analogue containing the paired substitutions Asn$^{B3}$→Lys and Lys$^{B29}$→Glu. A control analogue with native disulfide bridges was also prepared by chemical synthesis and chain combination.

An analogue of insulin *aspart* likewise containing pairwise substitution of Cys$^{A6}$ and Cys$^{A11}$ by Sec$^{A6}$ and Sec$^{A11}$ was prepared by chain combination essentially as described above in Example 2, except that the WT B chain (obtained from commercial insulin) was replaced by a synthetic analogue containing the paired substitutions Pro$^{B28}$→Asp. A control analogue with native disulfide bridges was also prepared by chemical synthesis and chain combination.

An analogue of insulin *glargine* likewise containing pairwise substitution of Cys$^{A6}$ and Cys$^{A11}$ by Sec$^{A6}$ and Sec$^{A11}$ was prepared by chain combination by a modified procedure. The reaction employed a variant [Sec$^{A6}$, Sec$^{A11}$, Gly$^{A21}$] A chain and variant B chain containing C-terminal extension Arg$^{B31}$-Arg$^{B32}$. Under the "standard" reaction conditions above, the yield was about threefold lower than that of chain combination reactions with the WT B chain, presumably due to the Arg$^{B31}$-Arg$^{B32}$ extension, which shifts the isoelectric point of the peptide and so its pH-dependent solubility. This effect was largely mitigated by raising the pH of the reaction from pH 10.6 to 11.2 (which enhanced the solubility of the variant B chain) and by extending the reaction time; in addition, the synthetic [C6U$^A$, C11U$^A$]-modified A chain was pre-dialyzed to remove ammonium salts prior to its use in the chain-combination reaction. This modified protocol enabled the efficiency of chain combination to be almost as high as was observed in the preparation of [C6U$^A$, C11U$^A$]-modified WT insulin. A control analogue with native disulfide bridges (*i.e.*, native insulin glargine) was obtained from commercial sources.

Four additional analogues of insulin *lispro* likewise containing pairwise substitution of Cys$^{A6}$ and Cys$^{A11}$ by Sec and further modified by either substitution Thr$^{A8}$→His, substitution Tyr$^{A14}$→Glu, paired substitutions [Thr$^{A8}$→His, Tyr$^{A14}$→Glu], or His$^{B10}$→Asp were prepared by chain combination essentially as described in Example 1 and listed in Table 1. Each of these four analogues of [C6U, C11U]-modified insulin *lispro* thus contains additional standard amino-acid substitutions that are known in the art to augment the thermodynamic stability of insulin.

**EXAMPLE 3**

Characterization of [C6U$^A$, C11U$^A$] Insulin Analogues

*Receptor-Binding Affinities.* Affinities of insulin or insulin analogues for the lectin-purified insulin receptor were evaluated using a microtiter plate antibody assay; representative studies of the [C6U$^A$, C11U$^A$] analogue of WT human insulin (Example 1 and 2 above) are shown in Fig. 7 in relation to Orn$^{B29}$-human insulin, a conservative analogue of human insulin. The assay exploited competitive displacement of $^{125}$I-Tyr$^{A14}$-insulin. Data were corrected for nonspecific binding (amount of radioactivity remaining membrane associated in the presence of 1 $\mu$M human insulin. Microtiter strip plates (Nunc Maxisorb) were incubated overnight at 4° C with AU5 IgG (100 $\mu$l/well of 40 mg/ml in phosphate-buffered saline). Binding data were analyzed by a two-site sequential model. Binding data were analyzed by non-linear regression using a heterologous competition model (Wang, Z.X. (1995) FEBS Lett. 360, 111-114) to obtain dissociation constants. In all assays the percentage of tracer bound in the absence of competing ligand was less than 15% to avoid ligand-depletion artifacts.

The affinities of the two insulin analogues containing paired Sec$^{A6}$ and Sec$^{A11}$ substitutions were indistinguishable from that of WT insulin or insulin *lispro* (see Fig. 7). Similar results were obtained on comparison of insulin *glulisine versus* insulin [C6U, C11U]-modified *glulisine* and on comparison of insulin *aspart versus* insulin [C6U, C11U]-modified *aspart.*

*Potency in Diabetic Rats.* The biological activities of insulin or insulin analogues were evaluated in Sprague-Dawley rats rendered diabetic through the action of beta-cell poison streptozotocin as described (Yang, Y., et al. (2010) J. Biol. Chem. 285: 10806-21). The rats exhibited a mean body mass of *ca.* 300 grams and a mean level of glycemia of *ca.* 400 mg/dL. Representative studies of the [C6U$^A$, C11U$^A$] analogue of insulin *lispro* (Example 2 above) are shown in Figs. 8A & 8B in relation to insulin *lispro;* their potencies (evaluated as the ability to reduce the blood-glucose concentration on subcutaneous injection) were indistinguishable. Similar results were obtained on comparative rat studies of insulin *glulisine versus* insulin [C6U, C11U]-modified *glulisine* and on comparison of insulin *aspart versus* insulin [C6U, C11U]-modified *aspart.*

The biological activities of representative insulin analogues in current clinical use were further evaluated in a human liver-derived cell line, HepG2 (hepatoma), chosen as a cell-culture model of the insulin-regulated signaling pathways in the liver. An assay of gene expression (quantitative real-time reverse-transcriptase polymerase chain reaction; q-RT-rtPCR) was employed to probe how the binding of insulin analogues to insulin receptors on the surface of these cells might alter the accumulation of mRNAs respectively encoding a key enzyme in the pathway of glucogeneosis (PEPCK) and key regulatory factors in the pathway of cholesterol or lipid synthesis (ChREBP and SREBP). In these assays the transcriptional regulatory activities of [C6U, C11U]-modified insulin *lispro* were indistinguishable from those of native insulin *lispro,* and similarly the transcriptional regulatory activities of [C6U, C11U]-modified insulin *glargine* were indistinguishable from those of native insulin *glargine* (Figs. 8C-8E).

*Mitogenicity.* An undesirable biological activity of an insulin analogue would be any increase in mitogenicity relative to WT insulin. Enhanced mitogenicity in the case of Asp$^{B10}$-insulin is known in the art to be associated with an excess of mammary tumors on long-term treatment of rats (Hansen, B.F., et al. (2011) Diabetologia 54: 2226-31). An assay for relative mitogenicity among insulin analogues is provided by transcriptional signaling leading to reciprocal regulation of the genes encoding cyclin D1 and cyclin G2 (Glidden, M.D., et al. (2018) J. Biol. Chem. 293: 47-68). As in the above studies of HepG2 cells in culture, an analogous q-RT-rtPCR assay was performed to probe mRNA accumulation in human breast-cancer cell line MCF-7; this cell line expresses the Type 1 IGF receptor (IGF-1R) as well as IR-A and IR-B. In this assay the signature of a mitogenic protein was provided by IGF-1 and Asp$^{B10}$-insulin. Treatment of MCF-7 cells with these proteins stimulates cyclin D1 transcription and inhibits cyclin G2 transcision to a more marked extent than does treatment with insulin lispro or Orn$^{B29}$-insulin (the latter contains conservative substitution Lys$^{B29}$→Orn and so provides a model of WT insulin). Results are summarized in gene-specific histograms (Fig. 9A, left) and are highlighted in a two-dimensional map (Fig. 9A, right). In these assays the mitogenic signature of [C6U, C11U]-modified insulin *lispro* was indistinguishable from that of native insulin *lispro,* and similarly the mitogenic signature of [C6U, C11U]-modified insulin *glargine* was indistinguishable from that of native insulin *glargine.* We note in passing that the *glargine* signature in both dimensions (*cyclin D1* activation and *cyclin* G2 repression) is intermediate between insulin *lispro* or Orn$^{B29}$-insulin and Asp$^{B10}$-insulin as is known in the art.

The studies of insulin signaling in MCF-7 breast-cancer cells were extended through Western blots to probe the extent of hormone-induced autophosphorylation of the insulin receptor (Fig. 9B, left) and hormone-induced downstream phosphorylation of the AKT kinase (Fig. 9B, right). In these assays the stimulatory activities of [C6U, C11U]-modified insulin *lispro* was indistinguishable from the stimulatory activities of native insulin *lispro;* the stimulatory activities of [C6U, C11U]-modified insulin *glargine* was indistinguishable from the stimulatory activities of native insulin *glargine;* the stimulatory activities of [C6U, C11U]-modified Des-Di (defined above) was indistinguishable from the stimulatory activities of native Des-Di; and the stimulatory activities of [C6U, C11U]-modified Orn[B29]-insulin was indistinguishable from the stimulatory activities of native Orn[B29]-insulin. In these assays insulin glargine and Asp[B10]-insulin exhibited greater activity than insulin *lispro* (or WT-like controls Des-Di and Orn[B29]-insulin) as is known in the art. The pairwise comparison of unmodified and [C6U, C11U]-modified analogues provides evidence that the diselenide bridge does not augment or attenuate hormone-triggered IR autophosphorylation or downstream AKT phosphorylation.

*Secondary Structure.* Far-ultraviolet circular dichroism (CD) spectra were obtained at 4° C and 25° C using an Aviv spectropolarimeter (Weiss et al. (2000) Biochemistry 39: 15429-15440). Samples contained *ca.* 25 $\mu$M WT insulin or analogues in 50 mM potassium phosphate (pH 7.4). Comparison of the spectra of WT human insulin and its [C6U[A], C11U[A]] analogue demonstrated similar features indicative of substantial alpha-helix content (Fig. 10A); the paired Sec substitutions were associated with a slight accentuation of alpha-helix content. Corresponding CD studies of insulin *lispro* and its [C6U[A], C11U[A]] analogue likewise demonstrated similar features but with more marked diselenide-associated accentuation (Fig. 10C). The latter difference represents a partial restoration of alpha-helix content that is perturbed in insulin *lispro* (relative to WT human insulin) due to its paired B-chain substitutions (Pro[B28]→Lys and Lys[B29]→Pro). The CD studies were repeated using a Jasco CD spectropolarimeter and extended to enable comparison of insulin *glulisine versus* its [C6U[A], C11U[A]] analogue and comparison of insulin *aspart versus* its [C6U[A], C11U[A]] analogue (Fig. 10E). Similar trends were in each case observed.

*Thermodynamic Stability.* Samples of insulin or insulin analogues were diluted to 5 $\mu$M for guanidine-induced denaturation studies at 25° C. To extract free energies of unfolding, denaturation transitions were fitted by non-linear least squares to a two-state model as described by Sosnick et al. (2000) Methods Enzymol. 317: 393-409. In brief, CD data $\theta(x)$, where x indicates the concentration of denaturant, were fitted by a nonlinear least-squares program according to

$$\theta(x) = \frac{\theta_A + \theta_B e^{(-\Delta G^o_{H_2O} - mx)/RT}}{1 + e^{-(\Delta G^o_{H_2O} - mx)/RT}}$$

where x is the concentration of guanidine and where $\theta_A$ and $\theta_B$ are baseline values in the native and unfolded states. Baselines were approximated by pre- and post-transition lines $\theta_A(x) = \theta_A^{H_2O} + m_A x$ and $\theta_B(x) = \theta_B^{H_2O} + m_B x$. The $m$ values obtained in fitting the variant unfolding transitions are lower than the m value obtained in fitting the wild-type unfolding curve. Such CD-monitored chemical denaturation studies (shown in Figs. 10B and 10D) demonstrated that the A6-A11 diselenide bridge augmented the stability of the parent analogues in Examples 1 and 2 (*i.e.*, WT human insulin and insulin *lispro,* respectively) by *ca.* 1 kcal/mole. This marked stabilization is similar in magnitude to that achieved by favorable amino-acid substitutions on the surface of the protein known in the art to stabilize the insulin molecule (Kaarsholm, N., et al. (1993) Biochemistry 32: 10773-8). The CD-detected guanidine-denaturation studies were repeated using a Jasco CD spectropolarimeter and extended to enable comparison of insulin *glulisine versus* its [C6U[A], C11U[A]] analogue and comparison of insulin *aspart versus* its [C6U[A], C11U[A]] analogue (Fig. 10F). Similar trends were in each case observed (Table 2). In addition, corresponding guanidine denaturation studies of the bottom four insulin analogues in Table 1, wherein the A6-A11 diselenide bridge was introduced in combination with a stabilizing standard amino-acid substitution, demonstrated that the thermodynamic effects were generally additive. This implies that the mechanism by which the diselenide bridge stabilizes the insulin monomer is complementary to and independent of that associated with standard substitutions on the surface of alpha-helices as known in the art: Asp[B10], His[A8] or Glu[A14]. These findings therefore provide evidence that the stability of an insulin analogue may be optimized by combination of standard amino-acid substitutions with the presently disclosed diselenide bridge.

CD-detected guanidine-denaturation studies were likewise performed at pH 4.0 (as in the pharmaceutical formulation of Lantus® and Toujeo®) to evaluate the effect of the A6-A11 diselenide brigde on the thermodynamic stability of insulin *glargine.* Whereas the parent analogue is less stable than WT insulin (presumably due in large part to the destabilizing Gly[A21] substitution), WT-like stability is restored by the paired Cys→Sec substitutions (Table 3).

*Resistance to Proteolysis by Glu-V8 protease.* To test whether the paired Sec substitutions might enhance the resistance of the insulin fold to peptide bond cleavage by Glu-V8 protease, stock solutions of WT human insulin and Se-insulin[C6U[A], C11U[A]] (1.0 mg/mL) were prepared in 0.1 M NH$_4$HCO$_3$ buffer and the protease was dissolved in water to obtain a concentration of 1.0 mg/mL. The reaction was initiated by adding 5 $\mu$L of the protease solution to a volume of 250 $\mu$L of insulin solution. The reaction was incubated at 37 °C. To monitor the degradation progress, 30 $\mu$L of the reaction aliquots were taken and quenched with 20 $\mu$L 0.1 % TFA in water and were placed in ice to stop the reaction. The aliquots were analyzed by analytical HPLC XSelect C18 column (3.5 $\mu$m, 130 Å, 4.6 × 150 mm) and eluted using a gradient of 10% B in eluent A for 2 min then 10-60% B over 20 min. The results demonstrated marked stabilization of the protein by the A6-A11 diselenide bridge in this assay (Fig. 11A).

*Resistance to reduction by glutathione (GSH).* To test whether the paired Sec substitutions protected the disulfide bridges and diselenide bridge in the folded state from reduction by GSH, stock solutions of WT human insulin and Se-insulin[C6U[A], C11U[A]] (1 mg/mL) were prepared in phosphate buffer (20 mM, pH 7.4, 0.1 M NaCl), and a stock solution of reduced glutathione was prepared in the same buffer (1.62 mM). The reaction was initiated by adding 92.6 μL of the GSH in 125 μL of protein solution; buffer was added to a volume of 500 μL. Concentration of insulin was 0.25 mg/mL and 300 μM for GSH. Denaturation was carried out at 37 °C. To monitor the reductive unfolding progress, 60 μL of the reaction aliquots were taken and quenched with 5 μL 0.1 % TFA in water. The aliquots were analyzed by analytical HPLC XSelect C4 column (3.5 μm, 130 Å, 4.6 × 150 mm) and eluted using a gradient of 5% B in eluent A for 2 min then 5-70% B over 20 min. The results demonstrated marked stabilization of the protein by the A6-A11 diselenide bridge in this assay (Fig. 11B).

*Resistance to onset of fibrillation.* To test whether the paired Sec substitutions protected the prandial insulin analogues in current clinical use (insulin *lispro,* insulin *glulisine* and insulin *aspart*) from fibrillation, lag times were measured as described (Vinther, T.N., et al. (2013) Protein Sci. 22: 296-305). These studies employed an instrument that vibrates a 96-well-plate under a defined temperature; the read-out was provided by Thioflavin T (ThT) flourescence. The results demonstrated marked stabilization of the protein by the A6-A11 diselenide bridge in this assay at 37 °C (Fig. 11C). Indeed, whereas the paired B-chain substitutions in insulin *lispro* (Pro[B28]→Lys and Lys[B29]→Pro) foreshorten the lag time, *i.e.,* hastening the onset of fibrillation as is known in the art, the A6-A11 diselenide bridge repairs this defect. The lag time of [C6U[A], C11U[A]]-repaired insulin *lispro* is in fact longer than that of WT human insulin; similar results were obtained in studies of insulin *aspart* and insulin *glulisine* (Table 2).

In these fibrillation assays WT insulin or analogues were made 60 μM in phosphate-buffered saline (pH 7.4) with 0.02% sodium azide and 16 μM ThT. Samples were plated in a Costar® plate (250 μl/well) and incubated at 37 °C with continuous shaking at 960 revolutions per minute in a Synergy H1M Biotek plate reader. ThT fluoresence at 480 nm (following excitation at 450 nm) was assessed at 15-minute intervals. The time of initial ThT fluorescence just prior to enhancement of ThT emission intensity was defined as *fibrillation lag time* as in known in the art.

**Table 2.** Thermodynamic and fibrillation studies of [C6U[A], C11U[A]]-modified insulin analogues

| Analog | $\Delta G_u$[a] *(kcal mol$^{-1}$)* | $C_{mid}$[b] *(M)* | $m$[c] *(kcal mol$^{-1}$ M$^{-1}$)* | $\Delta\Delta G_u$ *(kcal mol$^{-1}$)* | fibrillation lag times[d] *hours (N)* |
|---|---|---|---|---|---|
| human insulin | 3.6 ± 0.1 | 4.9 ± 0.1 | 0.74 ± 0.01 | | 4.93 ± 0.57 (10) |
| Se-human insulin | 4.4 ± 0.1 | 5.7 ± 0.1 | 0.77 ± 0.01 | 0.8 ± 0.1 | 9.56 ± 1.40 (8) |
| insulin *lispro* | 2.8 ± 0.1 | 4.8 ± 0.1 | 0.58 ± 0.01 | | 1.88 ± 0.27 (8) |
| Se-insulin *lispro* | 3.8 ± 0.1 | 5.4 ± 0.1 | 0.69 ± 0.01 | 1.0 ± 0.1 | 4.00 ± 0.69 (7) |
| *glulisine* | 2.9 ± 0.1 | 4.8 ± 0.1 | 0.61 ± 0.01 | | 1.38 ± 0.35 (8) |
| *Se-glulisine* | 4.3 ± 0.1 | 5.5 ± 0.1 | 0.78 ± 0.01 | 1.4 ± 0.1 | 3.59 ± 0.80 (8) |
| insulin *aspart* | 2.8 ± 0.1 | 4.8 ± 0.1 | 0.58 ± 0.01 | | 3.53 ± 0.40 (9) |
| *Se-insulin aspart* | 3.9 ± 0.1 | 5.6 ± 0.1 | 0.71 ± 0.01 | 1.1 ± 0.1 | 6.18 ± 0.79 (7) |

[a] Parameters were inferred from CD-detected guanidine denaturation data (Fig. 10F) by application of a two-state model; uncertainties represent fitting errors for a given data set.

[b] The *m*-value (slope $\Delta(G)/\Delta(M)$) correlates with extent of hydrophobic surfaces exposed on denaturation.

[c] $C_{mid}$ is the guanidine denaturant concentration at which 50% of the protein is in the unfolded state.

[d] Fibrillation lag times pertain to zinc-free insulin and analogs (in a monomer-dimer equilibrium); each protein was made 60 μM in phosphate-buffered saline (pH 7.4). Samples were agitated via continuous shaking at 37 °C. The time of initial Thioflavin T (ThT) fluorescence defined the lag time.

**Table 3.** Thermodynamic stabilities of pI-shifted insulin analogs at pH 4.0

| Analog | $\Delta G_u$[a] *(kcal mol$^{-1}$)* | $C_{mid}$[b] *(M)* | $m$[c] *(kcal mol$^{-1}$ M$^{-1}$)* | $\Delta\Delta G_u$ *(kcal mol$^{-1}$)* |
|---|---|---|---|---|
| human insulin | 3.8 ± 0.1 | 5.0 ± 0.1 | 0.76 ± 0.01 | |
| *Glargine* | 2.8 ± 0.1 | 4.9 ± 0.1 | 0.57 ± 0.01 | -1.0 ± 0.1 |
| Se-*glargine* | 3.8 ± 0.1 | 6.0 ± 0.1 | 0.63 ± 0.01 | +1.0 ± 0.1 |

[a] Parameters were inferred from CD-detected guanidine denaturation data by application of a two-state model; uncertainties represent fitting errors for a given data set.

[b] The *m*-value (slope $\Delta(G)/\Delta(M)$) correlates with extent of hydrophobic surfaces exposed on denaturation.

[c] $C_{mid}$ is the guanidine denaturant concentration at which 50% of the protein is in the unfolded state.

Table 4. Statistics pertaining to the solution structure of [Sec[A6], Sec[A11]]-insulin *lispro*

| parameter | SA ensemble [a] |
|---|---|
| RMSD values from experimental distance and dihedral-angle restraints | |
| all (866) | $0.038 \pm 0.002$ |
| intraresidue, i=j (285) | $0.044 \pm 0.003$ |
| sequential, \|i-j\|=1 (240) | $0.034 \pm 0.002$ |
| medium range, 1<\|i-j\|<5 (169) | $0.036 \pm 0.005$ |
| long range, \|i-j\|≥5 (172) | $0.032 \pm 0.004$ |
| dihedral angles (°, 77) | $0.96 \pm 0.07$ |
| RMSD values from idealized covalent geometry | |
| bonds (Å) | $0.0069 \pm 0.0008$ |
| angles (°) | $0.99 \pm 0.01$ |
| impropers (°) | $2.42 \pm 0.12$ |
| $E_{L-J}$ (kcal/mol) [b] | $-209.6 \pm 7.6$ |
| Coordinate precision of native insulin-like region [c] | |
| rmsd of backbone atoms to the mean (Å) | $0.28 \pm 0.06$ |
| rmsd of all heavy atoms to the mean (Å) | $0.93 \pm 0.16$ |

[a] Mean $\pm$ standard error where applicable.
[b] Lennard-Jones potential energy function, calculated with CHARMM19 (27) empirical energy parameters.
[c] Based on residues A2-A20 and B5-B26.

*2D-NMR studies of Se-insulin[C6U[A], C11U[A]] demonstrate native-like structure.* Samples of WT-insulin and Se-insulin[C6U[A], C11U[A]] were prepared under identical conditions. Proteins were dissolved in 286 μL distilled $H_2O$, and the pH was corrected to ~3.6 using solutions of NaOH 0.1 M and HCl 0.15 M. Acetonitrile-$d_3$ (ACN-$d_3$) was added to get a final ratio 65%:35%, $H_2O$:ACN-$d_3$. The final concentration of the Se-insulin[C6U[A], C11U[A]] was 0.93 mM and that of the WT-insulin 0.72 mM. NMR experiments were performed on a Bruker AVII 500 MHz spectrometer operating at the proton frequency of 500.13 MHz, using a 5-mm selective probe equipped with a self-shielded xyz-gradient coil at 27.2 °C. The transmitter frequency was set on the water signal and calibrated at 4.811 ppm. Correlation spectroscopy (COSY) experiments were acquired using gradients for water saturation under identical conditions. Spectra were processed and analyzed with TopSpin (Bruker Analytische Messtechnik GmbH) and SPARKY3 software. The resulting spectra (not shown) were similar to those of WT human insulin under the same conditions. Changes in $^1$H-NMR chemical shifts were observed near the sites of Sec substitution as expected.

*1D- and 2D-NMR studies of [C6U[A], C11U[A]]-modified insulin* lispro *imply native-like structure with a subtle dynamic change.* To test whether the A6-A11 diselenide bridge influences conformational fluctuations in an insulin monomer *in the absence of an organic cosolvent* (such as the 35% deuterated acetonitrile used above), $^1$H-NMR studies were undertaken of [C6U[A], C11U[A]]-"repaired" insulin *lispro* relative to insulin *lispro*. Spectra were obtained in 10 mM deuterioacetic acid at pH 3.0 in 90% $H_2O$ and 10% $D_2O$ as described (Hua, X.Q., *et al.* (2011) *Frontiers in Endocrinology* doi: 10.3389/fendo.2011.00048). One-dimensional (1D) spectra in the amide-aromatic region are similar but not identical (Figs. 12A and 12B); inset at upper left in the Fig. 12A are expansions of two amide downfield protons (9.4-9.8 ppm region) and two His $H_{\varepsilon}$ resonances (9.0-9.4 ppm region). These amide resonances are sharper in the [C6U[A], C11U[A]]-"repaired" insulin *lispro* (lower inset spectrum) relative to the corresponding resonances in the spectrum of insulin *lispro* (upper inset spectrum). The latter are broadened by millisecond motions on the intermediate NMR time scale. Such sharpening may be due to a change in time scale in either of two directions: faster or slower. Measurement of amide proton exchange (not shown) demonstrated slower exchange at this site, providing evidence of dynamic stabilization in accordance with the above four independent assays (chemical denaturation, resistance to proteolytic cleavage, resistance to reduction and resistance to fibrillation). Similar trends were observed on comparison of the $^1$H-NMR spectra under these conditions of insulin *glargine versus* [C6U[A], C11U[A]]-modified insulin *glargine* (Fig. 12C and 12D).

2D NMR studies of [C6U[A], C11U[A]]-modified insulin *lispro* under these conditions provided evidence for similar overall structures as indicated by nonlocal nuclear Overhauser effects (Fig 13A and 13C) and patterns of aromatic spin systems (four tyrosines and three phenylalanines; Figs. 13B and 13D). Complete resonance assignment and analysis of NOEs and a subset of J-coupling constants enabled calculation of an ensemble of solution structures (Fig. 13E). The solution structure of [C6U[A], C11U[A]]-modified insulin *lispro* is similar to that of unmodified insulin *lispro* as previously reported (Hua, X.Q., et al. (2011) Frontiers in Endocrinology doi: 10.3389/fendo.2011.00048).

*$^1$H-NMR-based Assessment of Thermodynamic Stability in $D_2O$.* The kinetics of $^1$H-$^2$H amide proton exchange can be used to measure the thermodynamic stability of a protein and to evaluate changes in such stability on modification. This method exploits amide protons whose exchange in $D_2O$ solution requires global unfolding of the protein. Application to insulin lispro has previously been described (Hua, X.Q., et al. (2011) Frontiers in Endocrinology doi: 10.3389/fendo.2011.00048). This experimental protocol pertains to solutions of insulin or insulin analogues at pD 3.0 (in a buffer containing 10 mM deuterated acetic acid) to avoid base-catalyzed amide-proton exchange and so make feasible the measurement of local exchange, subglobal exchange and global exchange kinetics on an accessible time scale. Comparison of the kinetics of $^1$H-$^2$H amide proton exchange in insulin *lispro versus* [C6U[A], C11U[A]]-modified insulin *lispro* under these conditions corroborated the overall results of the above CD-detected guanidine denaturation assays at neutral pH. 2D TOCSY spectra of residual cross peaks between main-chain amide resonances and carbon-bound resonances are shown at designated time points in [C6U[A], C11U[A]]-modified (Figs. 14A and 14C) *versus* unmodified insulin *lispro* (Figs. 14B and 14D). Comparison of two sites of global exchange in the respective A chains (residues Tyr[A19] and Leu[A16]) demonstrated in each case delayed exchange due to the A6-A11 diselenide bridge (Figs. 14E). Quantitative analysis of the degree of protection (the "protection factor") at each site indicated an increase in stability ($\Delta\Delta G_u$) of 0.4-0.5 kcal/mole under these conditions (pH 3.0 and 25 °C in a $D_2O$ solution). Although this gain in stability is less marked than that inferred above by CD-based guanidine denaturation experiments at neutral pH and in an $H_2O$ solution, the general trends are the same.

*X-ray crystallography.* To obtain single crystals suitable for X-ray diffraction, lyophilized human insulin containing the A6-A11 diselenide bridge was dissolved in 20 mM HCl to obtain an ~8 mg/mL protein solution, which was used subsequently for crystallization trials. Crystals, obtained through the hanging-drop vapor diffusion method, appeared after ~3 weeks in drops containing 2 µl protein and 1 µl reservoir, equilibrated over a 500 µl reservoir solution containing 1.5 M NaCl, 35 mM NaCitrate, 0.5 mM ZnAcetate, and 0.3 M Tris pH 7.5. The crystals were then soaked for ~10 sec in a cryogenic solution containing 20% glycerol and 80% reservoir solution prior to their flash-freezing in liquid nitrogen for X-ray diffraction data collection. X-ray diffraction data were collected at the Se peak ($\lambda$=0.976 Å, T=100 K) using a Pilatus 6M detector in the ID29 beamline of the ESRF synchrotron (Grenoble, France). The raw diffraction images were processed, integrated, and scaled with the XDS software, indicating a complete data set to 1.82 Å resolution belonging to the P1 space group. These data allowed the structure determination by the single anomalous dispersion (SAD) methodology, using the anomalous data that was obtained from the two Sec residues incorporated into the protein. The AutoSol software identified 20 Se atoms with a figure of merit of 0.379, producing phases of sufficient quality to automatically build 213 residues in the asymmetric unit, corresponding to respective $R_{work}$ and $R_{free}$ values of 36.00% and 37.84%. This initial structure was later on improved by iterative cycles of manual building with the program Coot, and concomitant refinement with the program phenix.refine. The final structure was shown to contain only 16 Se atoms, corresponding to 8 independent insulin analogue monomers in the asymmetric unit and 402 modeled protein residues, producing final $R_{work}$ and $R_{free}$ values of 18.96% and 22.92%, respectively. Geometric validation of the structure was conducted with Procheck and Molprobity, confirming a model of good structural and stereochemistry parameters, as demonstrated by root-mean-square deviation (RMSD) values of 0.013 Å and 1.491° for the bond lengths and bond angles, respectively. The final Se-Insulin structure was deposited in the RCSB protein data bank under accession code 6H3M. Representative electron density is shown in Fig. 15A; a representative molecule is shown as a ribbon diagram in Fig. 15B. The A6-A11 disulfide bridge is formed in the core at a position and within a structural environment similar to that of the native disulfide bridge in WT insulin. The secondary structure of this molecule is identical to that of WT position; the A20-B19 and A7-B7 disulfide bridges are essentially identical to the corresponding bridges in WT insulin.

*Kinetics of insulin hexamer disassembly.* The prandial insulin analogues in current clinical use exhibit more rapid absorption from a SQ depot. This property, critical to their therapeutic benefit, is due to more rapid dissociation of insulin hexamers relative to WT human insulin. It is therefore critical to evaluate whether the augmented stability of [C6U[A], C11U[A]]-modified prandial analogs might delay their dissociation from a hexameric assembly and thereby compromise their clinical efficacy as rapid-acting formulations. A model was provided by $R_6$ metal-ion-stabiized insulin hexamers wherein Co[2+] ions were substituted for the two axial zinc ions (Fig. 16A). This assay exploits the visible absorption spectrum of the tetrahedrally coordinated metal ion, which in the case of Co[2+] contains an incomplete d-electronic shell and so exhibits d-d optical transitions (Fig. 16A) as described (Rege, N., et al. (2018) J. Biol. Chem. 293: 10895-10910). The kinetic assay is triggered by addition of a high concentration of ethylenediamine-tetra-acetic acid (EDTA) to sequester the metal ions on release from a dissociated insulin hexamer. Because the competing Co[2+]/EDTA complex is octahedral (and not tetrahedral) the d-d transitions are negligible; and because the afiinity of Co[2+] for EDTA is much stronger than its affinity for insulin, addition of excess EDTA leads to sequestration of Co[2+] that is essentially irreversible. Baseline kinetic studies of the unmodified insulin analogs are shown in Fig. 16C, and companion studies of the [C6U[A], C11U[A]]-modified prandial analogs in Fig. 16D. Remarkably, the diselenide bridge is associated with accelerated hexamer diassembly despite its general stabilizing effect on the insulin analogue monomer. Mono-exponential curve fitting of these kinetic transitions indicates that the A6-A11 diselenide bridge leads, in each pair of cognate analogues (*e.g.*, insulin *lispro versus* [C6U[A], C11U[A]]-modified insulin *lispro*), to a *ca.* twofold reduction in estimated half-life at 25 °C.

The present modification of insulin and known insulin analogues provides a method to enhance the speed and fidelity of insulin chain combination, a reaction known in the art leading to specific disulfide pairing between the A- and B chains of insulin (Fig. 5). Because this method employs modification of only one of the two chains employed in this reaction (Fig. 6), this scheme represents a significant improvement relative to alternative strategies that would require substitution of selenocysteine in each of the two chains (Arai, K., *et al.* 2017). Further, the utility of exploiting an internal bridge (as distinct from an external bridge) is surprising given that the Selenium atom is larger than the Sulfur atom and given that the Se-Se bond is longer than the S-S bond-thereby in principle introducing a steric perturbation into the hydrophobic core of a globular protein. The present disclosure is also unrelated to the prior art by which the efficiency of insulin chain combination has been enhanced through use of a single-chain precursor (Zaykov, A.N., *et al.,* 2014) or A4-B30 side-chain ester linkage (Sohma, Y., and Kent, S. B., 2009). Whereas the paired B-chain substitutions in insulin *lispro* are known in the art to impair the efficiency of insulin chain combination, we have demonstrated that this perturbation can be mitigated and indeed overcome via introduction of selenocysteines at positions A6 and A11.

Although the Embodiments presented herein as examples were obtained by chemical synthesis, it is envisioned that the insulin analogues of the present disclosure may also be derived from Lys-directed proteolysis of a precursor polypeptide in yeast biosynthesis in *Pichia pastoris*, *Saccharomyces cerevisciae*, or other yeast expression species or strains. Such strains may be engineered to insert Sec at positions A6 and A11 by means of an engineered tRNA synthetase and orthogonal nonsense suppression.

**Claims**

1. An insulin analogue comprising an insulin A chain and an insulin B chain wherein

   said A chain comprises a sequence of $GIVEQX_6CX_8X_9IX_{11}SLYQLENYCX_{21}$-$R_{53}$ (SEQ ID NO: 17); and
   said B chain comprises the sequence
   $FVX_{23}QX_{25}LCGX_{29}X_{30}LVX_{33}X_{34}LYLVCGX_{41}X_{42}GFX_{45}$ (SEQ ID NO: 18), wherein

   $X_6$ is seleno-cysteine;
   $X_8$ is histidine, threonine, lysine, arginine, or tryptophan;
   $X_9$ is serine, lysine, or alanine;
   $X_{11}$ is seleno-cysteine;
   $X_{21}$ is selected from the group consisting of alanine, glycine, serine, threonine, glutamine, asparagine, and aspartic acid
   $X_{23}$ is Asn or Lys;
   $X_{25}$ is histidine, arginine or threonine;
   $X_{29}$ is selected from the group consisting of alanine, glycine and serine;
   $X_{30}$ is selected from the group consisting of histidine, aspartic acid, glutamic acid, homocysteic acid and cysteic acid;
   $X_{33}$ is selected from the group consisting of aspartic acid and glutamic acid;
   $X_{34}$ is selected from the group consisting of alanine and threonine;
   $X_{41}$ is selected from the group consisting of glutamic acid, aspartic acid and asparagine; $X_{42}$ is selected from the group consisting of alanine, ornithine, lysine and arginine;
   $X_{45}$ is tyrosine or phenylalanine; and
   $R_{53}$ is COOH or $CONH_2$.

2. The insulin analogue of claim 1 wherein said A chain comprises the sequence of $GIVEQX_6CX_8SIX_{11}SLYQLE$-$NYCX_{21}$-$R_{53}$ (SEQ ID NO: 15), and said B chain comprises the sequence $FVX_{23}QX_{25}LCGSHLVEALYLVCGERGF$-$FYTX_{48}X_{49}X_{50}$ (SEQ ID NO: 16), wherein

   $X_6$ is selenocysteine;
   $X_8$ is histidine, arginine or threonine;
   $X_{11}$ is selenocysteine;
   $X_{21}$ is alanine, glycine or asparagine;
   $X_{23}$ is Asn or Lys;
   $X_{25}$ is histidine or threonine;
   $X_{48}$ is proline, lysine or aspartic acid;
   $X_{49}$ is proline, lysine or glycine;
   $X_{50}$ is threonine, alanine or serine; and

$R_{53}$ is COOH or $CONH_2$.

3. The insulin analogue of claim 1 wherein the B chain sequence comprises the sequence FVKQX$_{25}$LCGSHLVEA-LYLVCGERGFF-R$_{63}$ (SEQ ID NO: 21), or FVNQX$_{25}$LCGSHLVEALYLVCGERGFF-R$_{63}$ (SEQ ID NO: 20), wherein

   $X_{25}$ is selected from the group consisting of histidine and threonine; and
   $R_{63}$ is selected from the group consisting of YTX$_{28}$KT (SEQ ID NO: 22), YTKPT (SEQ ID NO: 23), YTX$_{28}$K (SEQ ID NO: 24), YTKP (SEQ ID NO: 25), YTPK (SEQ ID NO: 26), YTX$_{28}$, YT, Y and a bond, wherein $X_{28}$ is proline, lysine, aspartic acid or glutamic acid.

4. The insulin analogue of claim 3 wherein the B chain sequence comprises the sequence FVKQX$_{25}$LCGSHLVEA-LYLVCGERGFFYTEKT (SEQ ID NO: 28),

   FVNQX$_{25}$LCGSHLVEALYLVCGERGFFYTDKT (SEQ ID NO: 27),
   FVNQX$_{25}$LCGSHLVEALYLVCGERGFFYTKPT (SEQ ID NO: 29) or
   FVNQX$_{25}$LCGSHLVEALYLVCGERGFFYTPKT (SEQ ID NO: 30) wherein
   $X_{25}$ is selected from the group consisting of histidine and threonine, optionally wherein $X_{25}$ is histidine.

5. The insulin analogue of claim 1 wherein the A chain comprises a sequence GIVEQX$_6$CTSIX$_{11}$SLYQLENYCN (SEQ ID NO: 30) and said B chain comprises a sequence selected from the group consisting of FVNQHLCGSHLVEALYLVCGERGFFYTPKT (SEQ ID NO: 4), FVNQHLCGSHLVEALYLVCGERGFFYTDKT (SEQ ID NO: 5),

   FVNQHLCGSHLVEALYLVCGERGFFYTKPT (SEQ ID NO: 6),
   FVKQHLCGSHLVEALYLVCGERGFFYTPET (SEQ ID NO: 7),
   FVNQHLCGSHLVEALYLVCGERGFFYTPKTRR (SEQ ID NO: 8), and
   FVNQHLCGSHLVEALYLVCGERGFFYTPX$_{29}$X$_{30}$ (SEQ ID NO: 9), wherein

   $X_6$ is selenocysteine;
   $X_{11}$ is selenocysteine;
   $X_{29}$ is an acyl derivative of lysine or glutamic-acid-bridged acyl derivative of lysine, and
   $X_{30}$ is Thr, Ala, Ser or absent.

6. The insulin analogue of claim 1 wherein the A chain comprises the amino acid sequence GIVEQX$_6$CTSIX$_{11}$SLYQ-LENYCN-R$_{53}$ (SEQ ID NO: 30) and the B chain comprises the sequence FVNQHLCGSHLVEALYLVCGERGFFYTPKT (SEQ ID NO: 4), wherein

   $X_6$ is selenocysteine;
   $X_{11}$ is selenocysteine and
   $R_{53}$ is COOH or $CONH_2$.

7. The insulin analogue of claim 4 wherein said A chain comprises the sequence GIVEQX$_6$CTSIX$_{11}$SLYQLENYCN-R$_{53}$ (SEQ ID NO: 30) and said B chain comprises the sequence FVKQX$_{25}$LCGSHLVEALYLVCGERGFFYTEKT (SEQ ID NO: 28) or FVNQX$_{25}$LCGSHLVEALYLVCGERGFFYTKPT (SEQ ID NO: 29), wherein

   $X_{25}$ is histidine; and
   $R_{53}$ is COOH or $CONH_2$.

8. The insulin analogue of any one of claims 1-7 wherein a hydrophilic moiety is covalently linked at one or more positions corresponding to A14, A15, B0, B1, B10, B22, B28, B29.

9. The insulin analogue of claim 8, wherein the hydrophilic moiety is a polyethylene glycol.

10. The insulin analogue of any one of claims 1-9, further comprising an acyl group or alkyl group covalently linked to an amino-acid side chain of said insulin analog.

11. The insulin analogue of claim 10 wherein said acyl group or alkyl group is covalently linked to one or more positions selected from A14, A15, B0, B1, B10, B22, B28, B29 of the insulin peptide.

**12.** A non-covalent dimer or multimer comprising two or more insulin analogues of any one of claims 1-11.

**13.** A pharmaceutical composition comprising an insulin analogue of any one of claims 1-12, or pharmaceutically acceptable salt thereof, and a pharmaceutically acceptable carrier.

**14.** A pharmaceutical composition of claim 13 wherein the augmented intrinsic stabilities of the insulin analogues of claims 1-12 make unnecessary the inclusion of zinc ions in the formulation and so enable inclusion of excipients associated with rapid absorption of insulin analogues from a subcutaneous depot, but that would otherwise sequester zinc ions, such as triphosphate ions, trisulphate ions, and chelating agents, including (but not limited to) ethylenediamine-tetra-acetic acid (EDTA) and ethylene glycol-bis(β-aminoethyl ether)-N,N,N',N'-tetraacetic acid (EGTA).

**15.** The insulin analogue of any one of claims 1-12 for use in the treatment of diabetes.

**Patentansprüche**

**1.** Insulinanalogon, umfassend eine Insulin-A-Kette und eine Insulin-B-Kette, wobei

die A-Kette eine Sequenz $GIVEQX_6CX_9X_9IX_{11}SLYQLENYCX_{21}$-$R_{53}$ (SEQ ID NO: 17) umfasst; und
die B-Kette die Sequenz $FVX_{23}QX_{25}LCGX_{29}X_{30}LVX_{33}X_{34}LYLVCGX_{41}X_{42}GFX_{45}$ (SEQ ID NO: 18) umfasst, wobei
$X_6$ Selenocystein ist;
$X_8$ Histidin, Threonin, Lysin, Arginin oder Tryptophan ist;
$X_9$ Serin, Lysin oder Alanin ist;
$X_{11}$ Selenocystein ist;
$X_{21}$ ausgewählt ist aus der Gruppe bestehend aus Alanin, Glycin, Serin, Threonin, Glutamin, Asparagin und Asparaginsäure
$X_{23}$ Asn oder Lys ist;
$X_{25}$ Histidin, Arginin oder Threonin ist;
$X_{29}$ ausgewählt ist aus der Gruppe bestehend aus Alanin, Glycin und Serin;
$X_{30}$ ausgewählt ist aus der Gruppe bestehend aus Histidin, Asparaginsäure, Glutaminsäure, Homocysteinsäure und Cysteinsäure;
$X_{33}$ ausgewählt ist aus der Gruppe bestehend aus Asparaginsäure und Glutaminsäure;
$X_{34}$ ausgewählt ist aus der Gruppe bestehend aus Alanin und Threonin;
$X_{41}$ ausgewählt ist aus der Gruppe bestehend aus Glutaminsäure, Asparaginsäure und Asparagin;
$X_{42}$ ist ausgewählt aus der Gruppe bestehend aus Alanin, Ornithin, Lysin und Arginin;
$X_{45}$ Tyrosin oder Phenylalanin ist; und
$R_{53}$ COOH oder $CONH_2$ ist.

**2.** Insulinanalogon nach Anspruch 1, wobei die A-Kette die Sequenz $GIVEQX_6CX_8SIX_{11}SLYQLENYCX_{21}$-$R_{53}$ (SEQ ID NO: 15) umfasst und die B-Kette die Sequenz $FVX_{23}QX_{25}LCGSHLVEALYLVCGERGFFYTX_{48}X_{49}X_{50}$ (SEQ ID NO: 16) umfasst, wobei

$X_6$ Selenocystein ist;
$X_8$ Histidin, Arginin oder Threonin ist;
$X_{11}$ Selenocystein ist;
$X_{21}$ Alanin, Glycin oder Asparagin ist;
$X_{23}$ Asn oder Lys ist;
$X_{25}$ Histidin oder Threonin ist;
$X_{48}$ Prolin, Lysin oder Asparaginsäure ist;
$X_{49}$ Prolin, Lysin oder Glycin ist;
$X_{50}$ Threonin, Alanin oder Serin ist; und
$R_{53}$ COOH oder $CONH_2$ ist.

**3.** Insulinanalogon nach Anspruch 1, wobei die B-Kettensequenz die Sequenz $FVKQX_{25}LCGSHLVEALYLVC$-$GERGFF$-$R_{63}$ (SEQ ID NO: 21) oder $FVNQX_{25}LCGSHLVEALYLVCGERGFF$-$R_{63}$ (SEQ ID NO: 20) umfasst, wobei

$X_{25}$ ausgewählt ist aus der Gruppe bestehend aus Histidin und Threonin; und

$R_{63}$ ausgewählt ist aus der Gruppe bestehend aus YTX$_{28}$KT (SEQ ID NO: 22), YTKPT (SEQ ID NO: 23), YTX$_{28}$K (SEQ ID NO: 24), YTKP (SEQ ID NO: 25), YTPK (SEQ ID NO: 26), YTX$_{28}$, YT, Y und einer Bindung, wobei $X_{28}$ Prolin, Lysin, Asparaginsäure oder Glutaminsäure ist.

4. Insulinanalogon nach Anspruch 3, wobei die B-Kettensequenz die Sequenz FVKQX$_{25}$LCGSHLVEALYLVCGERGF-FYTEKT (SEQ ID NO: 28) umfasst,

FVNQX$_{25}$LCGSHLVEALYLVCGERGFFYTDKT (SEQ ID NO: 27),
FVNQX$_{25}$LCGSHLVEALYLVCGERGFFYTKPT (SEQ ID NO: 29) oder
FVNQX$_{25}$LCGSHLVEALYLVCGERGFFYTPKT (SEQ ID NO: 30), wobei
$X_{25}$ ausgewählt ist aus der Gruppe bestehend aus Histidin und Threonin, wobei $X_{25}$ optional Histidin ist.

5. Insulinanalogon nach Anspruch 1, wobei die A-Kette eine Sequenz GIVEQX$_6$CTSIX$_{11}$SLYQLENYCN (SEQ ID NO: 30) umfasst und die B-Kette eine Sequenz umfasst, die ausgewählt ist aus der Gruppe bestehend aus

FVNQHLCGSHLVEALYLVCGERGFFYTPKT (SEQ ID NO: 4),
FVNQHLCGSHLVEALYLVCGERGFFYTDKT (SEQ ID NO: 5),
FVNQHLCGSHLVEALYLVCGERGFFYTKPT (SEQ ID NO: 6),
FVKQHLCGSHLVEALYLVCGERGFFYTPET (SEQ ID NO: 7),
FVNQHLCGSHL VEAL YL VCGERGFFYTPKTRR (SEQ ID NO: 8) und
FVNQHLCGSHLVEALYLVCGERGFFYTPX$_{29}$X$_{30}$ (SEQ ID NO: 9), wobei

$X_6$ Selenocystein ist;
$X_{11}$ Selenocystein ist;
$X_{29}$ ein Acylderivat von Lysin oder ein mit Glutaminsäure gebrücktes Acylderivat von Lysin ist und $X_{30}$ Thr, Ala, Ser ist oder fehlt.

6. Insulinanalogon nach Anspruch 1, wobei die A-Kette die Aminosäuresequenz GIVEQX$_6$CTSIX$_{11}$SLYQLENYCN-R$_{53}$ (SEQ ID NO: 30) umfasst und die B-Kette die Sequenz FVNQHLCGSHLVEALYLVCGERGFFYTPKT (SEQ ID NO: 4) umfasst, wobei

$X_6$ Selenocystein ist;
$X_{11}$ Selenocystein ist und
$R_{53}$ COOH oder CONH$_2$ ist.

7. Insulinanalogon nach Anspruch 4, wobei die A-Kette die Sequenz GIVEQX$_6$CTSIX$_{11}$SLYQLENYCN-R$_{53}$ (SEQ ID NO: 30) umfasst und die B-Kette die Sequenz FVKQX$_{25}$LCGSHLVEALYLVCGERGFFYTEKT (SEQ ID NO: 28) oder FVNQX$_{25}$LCGSHLVEALYLVCGERGFFYTKPT (SEQ ID NO: 29) umfasst, wobei

$X_{25}$ Histidin ist; und
$R_{53}$ COOH oder CONH$_2$ ist.

8. Insulinanalogon nach einem der Ansprüche 1-7, wobei eine hydrophile Einheit an einer oder mehreren Positionen, die A14, A15, B0, B1, B10, B22, B28, B29 entsprechen, kovalent verknüpft ist.

9. Insulinanalogon nach Anspruch 8, wobei die hydrophile Einheit ein Polyethylenglykol ist.

10. Insulinanalogon nach einem der Ansprüche 1-9, ferner umfassend eine Acylgruppe oder Alkylgruppe, die kovalent mit einer Aminosäureseitenkette des Insulinanalogons verknüpft ist.

11. Insulinanalogon nach Anspruch 10, wobei die Acylgruppe oder Alkylgruppe kovalent mit einer oder mehreren Positionen, die aus A14, A15, B0, B1, B10, B22, B28, B29 des Insulinpeptids ausgewählt sind, verknüpft ist.

12. Nicht kovalentes Dimer oder Multimer, umfassend zwei oder mehr Insulinanaloga nach einem der Ansprüche 1-11.

13. Pharmazeutische Zusammensetzung, umfassend ein Insulinanalogon nach einem der Ansprüche 1-12 oder ein pharmazeutisch unbedenkliches Salz davon und einen pharmazeutisch unbedenklichen Träger.

**14.** Pharmazeutische Zusammensetzung nach Anspruch 13, wobei die erhöhten intrinsischen Stabilitäten der Insulinanaloga der Ansprüche 1-12 den Einschluss von Zinkionen in die Formulierung unnötig machen und so den Einschluss von Hilfsstoffen ermöglichen, die mit einer schnellen Absorption von Insulinanaloga aus einem subkutanen Depot verbunden sind, die aber ansonsten Zinkionen sequestrieren würden, wie Triphosphationen, Trisulfationen und Chelatbildner, einschließlich (aber nicht beschränkt auf) Ethylendiamintetraessigsäure (EDTA) und Ethylenglykol-bis($\beta$-aminoethylether)-N,N,N',N'-tetraessigsäure (EGTA).

**15.** Insulinanalogon nach einem der Ansprüche 1-12 zur Verwendung bei der Behandlung von Diabetes.

**Revendications**

**1.** Analogue d'insuline comprenant une chaîne A d'insuline et une chaîne B d'insuline,

ladite chaîne A comprenant une séquence de $GIVEQX_6CX_8X_9IX_{11}SLYQLENYCX_{21}$-$R_{53}$ (SEQ ID n°: 17) ; et
ladite chaîne B comprenant la séquence
$FVX_{23}QX_{25}LCGX_{29}X_{30}LVX_{33}X_{34}LYLVCGX_{41}X_{42}GFX_{45}$ (SEQ ID n°: 18), dans lesquelles
$X_6$ représente la sélénocystéine ;
$X_8$ représente l'histidine, la thréonine, la lysine, l'arginine ou le tryptophane ;
$X_9$ représente la sérine, la lysine ou l'alanine ;
$X_{11}$ représente la sélénocystéine ;
$X_{21}$ est choisi dans le groupe constitué par l'alanine, la glycine, la sérine, la thréonine, la glutamine, l'asparagine et l'acide aspartique
$X_{23}$ représente Asn ou Lys ;
$X_{25}$ représente l'histidine, l'arginine ou la thréonine ;
$X_{29}$ est choisi dans le groupe constitué par l'alanine, la glycine et la sérine ;
$X_{30}$ est choisi dans le groupe constitué par l'histidine, l'acide aspartique, l'acide glutamique, l'acide homocystéique et l'acide cystéique ;
$X_{33}$ est choisi dans le groupe constitué par l'acide aspartique et l'acide glutamique ;
$X_{34}$ est choisi dans le groupe constitué par l'alanine et la thréonine ;
$X_{41}$ est choisi dans le groupe constitué par l'acide glutamique, l'acide aspartique et l'asparagine ;
$X_{42}$ est choisi dans le groupe constitué par l'alanine, l'ornithine, la lysine et l'arginine ;
$X_{45}$ représente la tyrosine ou la phénylalanine ; et
$R_{53}$ représente COOH ou $CONH_2$.

**2.** Analogue d'insuline selon la revendication 1, ladite chaîne A comprenant la séquence de $GIVEQX_6CX_8SIX_{11}S$-$LYQLENYCX_{21}$-$R_{53}$ (SEQ ID n°: 15), et ladite chaîne B comprenant la séquence $FVX_{23}QX_{25}LCGSHLVEALYLVC$-$GERGFFYTX_{48}X_{49}X_{50}$ (SEQ ID n°: 16), dans lesquelles

$X_6$ représente la sélénocystéine ;
$X_8$ représente l'histidine, l'arginine ou la thréonine ;
$X_{11}$ représente la sélénocystéine ;
$X_{21}$ représente l'alanine, la glycine ou l'asparagine ;
$X_{23}$ représente Asn ou Lys ;
$X_{25}$ représente l'histidine ou la thréonine ;
$X_{48}$ représente la proline, la lysine ou l'acide aspartique ;
$X_{49}$ représente la proline, la lysine ou la glycine ;
$X_{50}$ représente la thréonine, l'alanine ou la sérine ; et
$R_{53}$ représente COOH ou $CONH_2$.

**3.** Analogue d'insuline selon la revendication 1, ladite séquence de la chaîne B comprenant la séquence $FVKQX_{25}LCGSHLVEALYLVCGERGFF$-$R_{63}$ (SEQ ID n°: 21), ou $FVNQX_{25}LCGSHLVEALYLVCGERGFF$-$R_{63}$ (SEQ ID n°: 20), dans lesquelles

$X_{25}$ est choisi dans le groupe constitué par l'histidine et la thréonine ; et
$R_{63}$ est choisi dans le groupe constitué par $YTX_{28}KT$ (SEQ ID n°: 22), YTKPT (SEQ ID n°: 23), $YTX_{28}K$ (SEQ ID n°: 24), YTKP (SEQ ID n°: 25), YTPK (SEQ ID n°: 26), $YTX_{28}$, YT, Y et une liaison, où $X_{28}$ représente la proline, la lysine, l'acide aspartique ou l'acide glutamique.

4. Analogue d'insuline selon la revendication 3, ladite séquence de la chaîne B comprenant la séquence FVKQX$_{25}$LCGSHLVEALYLVCGERGFFYTEKT (SEQ ID n°: 28),

> FVNQX$_{25}$LCGSHLVEALYLVCGERGFFYTDKT (SEQ ID n°: 27),
> FVNQX$_{25}$LCGSHLVEALYLVCGERGFFYTKPT (SEQ ID n°: 29) ou
> FVNQX$_{25}$LCGSHLVEALYLVCGERGFFYTPKT (SEQ ID n°: 30), dans lesquelles
> X$_{25}$ est choisi dans le groupe constitué par l'histidine et la thréonine, éventuellement X$_{25}$ représentant l'histidine.

5. Analogue d'insuline selon la revendication 1, ladite chaîne A comprenant une séquence GIVEQX$_6$CTSIX$_{11}$SLYQ-LENYCN(SEQ ID n°: 30) et ladite chaîne B comprenant une séquence choisie dans le groupe constitué par FVNQHLCGSHLVEALYLVCGERGFFYTPKT (SEQ ID n°: 4), FVNQHLCGSHLVEALYLVCGERGFFYTDKT (SEQ ID n°: 5),

> FVNQHLCGSHLVEALYLVCGERGFFYTKPT(SEQ ID n°: 6),
> FVKQHLCGSHLVEALYLVCGERGFFYTPET (SEQ ID n°: 7),
> FVNQHLCGSHLVEALYLVCGERGFFYTPKTRR(SEQ ID n°: 8), et
> FVNQHLCGSHLVEALYLVCGERGFFYTPX$_{29}$X$_{30}$(SEQ ID n°: 9), dans lesquelles

>> X$_6$ représente la sélénocystéine ;
>> X$_{11}$ représente la sélénocystéine ;
>> X$_{29}$ représente un dérivé acyle de la lysine ou un dérivé acyle de la lysine ponté par l'acide glutamique, et
>> X$_{30}$ représente Thr, Ala, Ser ou est absent.

6. Analogue d'insuline selon la revendication 1, ladite chaîne A comprenant la séquence d'acides aminés GIVEQX$_6$CT-SIX$_{11}$SLYQLENYCN-R$_{53}$ (SEQ ID n°: 30) et ladite chaîne B comprenant la séquence FVNQHLCGSHLVEALYLVCGERGFFYTPKT (SEQ ID n°: 4), dans lesquelles

> X$_6$ représente la sélénocystéine ;
> X$_{11}$ représente la sélénocystéine et
> R$_{53}$ représente COOH ou CONH$_2$.

7. Analogue d'insuline selon la revendication 4, ladite chaîne A comprenant la séquence GIVEQX$_6$CTSIX$_{11}$SLYQLE-NYCN-R$_{53}$ (SEQ ID n°: 30) et ladite chaîne B comprenant la séquence FVKQX$_{25}$LCGSHLVEALYLVCGERGFFY-TEKT (SEQ ID n°: 28) ou FVNQX$_{25}$LCGSHLVEALYLVCGERGFFYTKPT (SEQ ID n°: 29), dans lesquelles

> X$_{25}$ représente l'histidine ; et
> R$_{53}$ représente COOH ou CONH$_2$.

8. Analogue d'insuline selon l'une quelconque des revendications 1 à 7, un groupement hydrophile étant lié de manière covalente à une ou plusieurs positions correspondant à A14, A15, B0, B1, B10, B22, B28, B29.

9. Analogue d'insuline selon la revendication 8, ledit groupement hydrophile étant un polyéthylène glycol.

10. Analogue d'insuline selon l'une quelconque des revendications 1 à 9, comprenant en outre un groupe acyle ou un groupe alkyle lié de manière covalente à une chaîne latérale d'acides aminés dudit analogue d'insuline.

11. Analogue d'insuline selon la revendication 10, ledit groupe acyle ou groupe alkyle étant lié de manière covalente à une ou plusieurs positions choisies parmi A14, A15, B0, B1, B10, B22, B28, B29 du peptide d'insuline.

12. Dimère ou multimère non covalent comprenant deux analogues ou plus de l'insuline selon l'une quelconque des revendications 1 à 11.

13. Composition pharmaceutique comprenant un analogue d'insuline selon l'une quelconque des revendications 1 à 12, ou un sel pharmaceutiquement acceptable de celui-ci, et un vecteur pharmaceutiquement acceptable.

14. Composition pharmaceutique selon la revendication 13, les stabilités intrinsèques accrues des analogues de l'insuline des revendications 1 à 12 rendant inutile l'inclusion d'ions zinc dans la formulation et permettant ainsi l'inclusion d'excipients associés à l'absorption rapide d'analogues de l'insuline à partir d'un dépôt sous-cutané, mais qui, autrement, séquestreraient les ions zinc, tels que les ions triphosphate, les ions trisulfate et les agents chélatants, y compris (mais sans s'y limiter) l'acide éthylène-diamine-tétra-acétique (EDTA) et l'acide éthylène glycol-bis($\beta$-aminoéthyléther)-N,N,N',N'-tétra-acétique (EGTA).

15. Analogue d'insuline selon l'une quelconque des revendications 1 à 12 destiné à être utilisé dans le traitement du diabète.

**FIG. 1A**

**FIG. 1B**

**FIG. 1C**

pK$_a$ *ca.* 8.3
atomic radius S: 105 pm
E$_a$ = -220 mV

pK$_a$ *ca.* 5.2
atomic radius Se: 120 pm
E$_a$ = -388 mV

FIG. 2A

Se—Se   *bridge*

*polypeptide main chain (or chains)*

FIG. 2B

**FIG. 2C**

**FIG. 3**

**FIG. 4**

**FIG. 5**

**FIG. 6**

**FIG. 7**

**FIG. 8A**

**FIG. 8B**

HepG2

**FIG. 8C**

**FIG. 8D**

FIG. 8E

FIG. 9A

FIG. 9B

**FIG. 10A**

**FIG. 10B**

FIG. 10C

FIG. 10D

**FIG. 10E**

**FIG. 10F**

**FIG. 11A**

**FIG. 11B**

**FIG 11C**

Amide and aromatic region of ¹H-NMR spectrum

**FIG. 12A**

Se-KP

Amide and aromatic region of $^1$H-NMR spectrum

**FIG. 12B**

**FIG. 12C** insulin *glargine*

**FIG. 12D** Se-*glargine*

NOESY

**FIG. 13A**

TOCSY

KP control

**FIG. 13B**

NOESY

**FIG. 13C**

TOCSY

Se-KP

**FIG. 13D**

**FIG. 13E**

**FIG. 14A**

**FIG. 14B**

**FIG. 14C**

**FIG. 14D**

FIG. 14E

FIG. 14F

**FIG. 15A**

**FIG. 15B**

**FIG. 16A**

**FIG. 16B**

**FIG. 16C**

**FIG. 16D**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9634882 A

- US 6630348 B

**Non-patent literature cited in the description**

- **ALTSCHUL et al.** Basic Local Alignment Search Tool. *J. Mol. Biol.*, 1993, vol. 215, 403-410
- **MERRIFIELD et al.** *Biochemistry*, 1982, vol. 21, 5020-5031
- **ZYKOV, A.N. et al.** *ACS Chem. Biol.*, 2014, vol. 9, 683-91
- **KUBIAC, T.** ; **COWBURN, D.** *Int. J. Pept. Protein Res.*, 1986, vol. 27, 514-21
- **WANG, Z.X.** *FEBS Lett.*, 1995, vol. 360, 111-114
- **YANG, Y. et al.** *J. Biol. Chem.*, 2010, vol. 285, 10806-21
- **HANSEN, B.F. et al.** *Diabetologia*, 2011, vol. 54, 2226-31
- **GLIDDEN, M.D. et al.** *J. Biol. Chem.*, 2018, vol. 293, 47-68
- **WEISS et al.** *Biochemistry*, 2000, vol. 39, 15429-15440
- **SOSNICK et al.** *Methods Enzymol.*, 2000, vol. 317, 393-409
- **KAARSHOLM, N. et al.** *Biochemistry*, 1993, vol. 32, 10773-8
- **VINTHER, T.N. et al.** *Protein Sci.*, 2013, vol. 22, 296-305
- **HUA, X.Q. et al.** *Frontiers in Endocrinology*, 2011
- **REGE, N. et al.** *J. Biol. Chem.*, 2018, vol. 293, 10895-10910